# EUROPEAN PATENT APPLICATION

(11) **EP 1 980 628 A2**
(43) Date of publication of application: **15.10.2008**
(21) Application number: 08005904.1
(22) Date of filing: 18.02.2005
(51) Int. Cl.: C12Q 1/68, G01N 33/50

(54) **Surrogate markers of neuropathic pain**

(30) Priority: 20.02.2004 US 784004
(62) Divisional of application: 05738782.1
(71) Applicant: Biogen Idec MA Inc., Cambridge, Massachusetts 02142 (US)
(72) Inventor: Sah, Dinah, W., Y., Boston MA Massachusetts 02114 (US); Cate, Richard, Cohasset MA Massachusetts 02025 (US); Ehrenfels, Christian, W., Chelmsford MA Massachusets 01824 (US); Szak, Suzanne, Arlington MA Massachusetts 02474 (US); Bandaru, Rjasekhar, Malden, MA 02148 (US)
(74) Representative: Pohlman, Sandra M.

(57) **Abstract**

The disclosure provides methods and compositions for the evaluation of neuropathic pain and neurotrophic or other activity of a drug or drug candidate. In the disclosed methods, expression of certain gene(s) in tissue extracts from skin biopsies serves as a proxy of a relevant endpoint.

## Description

### Field of the Invention

The invention is in the fields of neurology and pharmacology. The invention generally relates to methods of evaluating neuropathic pain and to methods of evaluating biological activity of drugs or drug candidates for treating neuropathies.

### Background of the Invention

Painful neuropathies are characterized by spontaneous and/or abnormal stimulus-evoked pain such as allodynia or hyperalgesia. Symptoms of neuropathic pain often include spontaneous cramping, burning, or shooting pain, or pain caused by normally innocuous stimuli. Neuropathic pain has a neurogenic origin, i.e., it is initiated or caused by a primary lesion or dysfunction in the peripheral or central nervous system (see, e.g., Merskey and Bogdik (1994) Classification of Chronic Pain: Descriptions of Chronic Pain Syndromes and Definitions of Pain Terms, 2nd ed., Seattle: IASP Press). Neuropathic pain can occur as a result of nerve damage due to infectious agents (e.g., herpesviruses), metabolic diseases (e.g., diabetes), neurodegenerative diseases (e.g., multiple sclerosis), nerve injury (e.g., amputation or cancer-induced nerve compression), etc. Current pharmacologic and nonpharmacologic therapies for chronic neuropathic pain provide only partial relief and the outcomes vary widely in individual patients.

Conditions affecting the peripheral nervous system create pathophysiologic changes such as loss of small sensory fibers and/or demyelination. Such changes can be histologically observed in the skin. Indeed, histological evaluation of skin biopsies has become an accepted method for assessing peripheral nerve status in patients with neuropathic pain or peripheral neuropathy (Griffin et al. (2001) Curr. Opin. Neurol., 14:655-659). This approach allows one to evaluate the progression of nerve damage in disease and regeneration/re-innervation with treatment. Counting criteria include epidermal nerve fiber density, the number of fibers crossing the dermal-epidermal junction, etc. Skin biopsies can be performed in multiple sites over time, so that a spatiotemporal profile of epidermal innervation can be assessed. However, histological analysis of skin biopsies is a laborious and time-consuming procedure.

Therefore, there exists a need in the art to develop new methods for treatment and assessment of neuropathic pain and peripheral neuropathy.

### SUMMARY OF THE INVENTION

The present invention results from the realization that skin biopsy samples can be nonhistologically evaluated for expression of gene(s) that reflect the neuropathic pain status ("surrogate markers of neuropathic pain," also known as "marker of injury/disease"). The expression of such genes can be measured in skin biopsy homogenates in a rapid and quantitative manner. If the expression of the gene(s) in skin punch biopsy samples correlates with the beneficial effect of the drug or drug candidate on neuropathic pain or peripheral neuropathy, then the read-out represents a surrogate marker of drug activity associated with the reduction in neuropathic pain and/or peripheral neuropathy ("surrogate marker of neurotrophic activity"). Furthermore, gene expression in skin punch biopsy samples can be used as a read-out of in vivo biological activity of a drug or drug candidate regardless of the neuropathic pain status ("biomarker of in vivo biological activity of a neurotrophic agent" or "biomarker of a neurotrophic agent" for short, also known as "marker of exposure to a neurotrophic agent").

In one aspect, the invention provides methods of identifying surrogate markers of neuropathic pain. The methods of identifying a surrogate marker of neuropathic pain include:
(a) obtaining a first skin biopsy sample under conditions of neuropathic pain;
(b) obtaining a second skin biopsy sample under conditions of substantially no neuropathic pain;
(c) preparing tissue extracts from the first and the second samples; and
(d) determining an amount of at least one nucleic acid or protein in the tissue extracts.
A difference between the amount of the nucleic acid or the protein in the first sample and the amount of the same nucleic acid or protein in the second sample indicates that the nucleic acid or the protein is a surrogate marker of neuropathic pain.

In another aspect, the invention provides methods of evaluating the level of neuropathic pain using such surrogate markers. The methods of evaluating the level of neuropathic pain using surrogate markers of neuropathic pain include:
(a) obtaining a first skin biopsy sample under conditions of neuropathic pain;
(b) obtaining a second skin biopsy sample under conditions of substantially no neuropathic pain;
(c) preparing tissue extracts from the first and the second samples; and
(d) determining an amount of at least one nucleic acid or protein in the tissues, the nucleic acid or the protein being a surrogate marker of neuropathic pain.
A difference between the amount of the nucleic acid or the protein in the first sample and the amount of the same nucleic acid or protein in the second sample indicates the level of neuropathic pain.

In another aspect, the invention provides methods of evaluating neurotrophic activity of a compound or composition, for example, in evaluating the effect of a compound of composition on the level of neuropathic pain. The methods include:
(a) administering the compound or composition to the mammal having neuropathic pain;
(b) obtaining at least one skin biopsy sample from the mammal;
(c) preparing a tissue extract from the skin biopsy sample; and
(d) determining an amount of at least of one surrogate marker of neuropathic pain that is nucleic acid or protein in the tissue extract.
A difference in the amount of the nucleic add or protein determined in step (d) and the amount of the same nucleic acid or protein expressed in the absence of the compound or composition i indicates the level of efficacy of the compound or composition on neuropathic pain.

In another aspect, the invention provides methods of identifying biomarkers of in vivo biological activity of a neurotrophic agent and methods of evaluating in vivo biological act ivity of a neurotrophic agent using such biomarkers. The methods of identifying biomarkers of in vivo biological activity of a neurotrophic agent include:
(a) administering the agent to a mammal;
(b) obtaining at least one skin biopsy sample from the mammal;
(c) preparing a tissue extract from the skin biopsy sample; and
(d) determining an amount of at least one nucleic acid or protein in the tissue extract.
A difference in the amount of the nucleic acid or protein determined in step (d) and the amount of the same nucleic acid or protein expressed in the absence of the agent indicates that the nucleic acid or the protein is a biomarker of in vivo biological activity of the agent.

In another aspect, the invention provides methods of evaluating in vivo biological activity of a neurotrophic agent using biomarkers of in vivo biological activity of such an agent. The methods of evaluating in vivo biological activity of a neurotrophic agent include:
(a) administering the agent to a mammal;
(b) obtaining at least one skin biopsy sample from the mammal;
(c) preparing a tissue extract from the skin biopsy sample; and
(d) determining an amount of at least one nucleic acid or protein in the tissue extract.
A difference in the amount of the nucleic acid or protein determined in step (d) and the amount of the same nucleic acid or protein expressed in the absence of the agent indicates that the agent is biologically active.

In illustrative embodiments, the neurotrophic agent being evaluated is artemin (also known as neublastin or enovin), a member of the glial-cell-line-derived neurotrophic factor (GDNF) family.

Exemplary nucleotide and/or amino acid sequences of human and rat surrogate markers of neuropathic pain, surrogated markers of neurotrophic activity and biomarkers of in vivo biological activity of neurotrophic agents are also provided (see Tables 1 and 1a).

**Table 1.**

| **Group No.** | **SEQ ID NOs:** | **Preferred SEQ ID NOs:** | **Category*** | **Type** | **Species** | **Table No.** |
|---|---|---|---|---|---|---|
| I | 1-308 | 1-42 | SMP | DNA** | Rat | 2 |
| II | 309-470 | 309-333 | SMP | Protein | Rat | 3 |
| III | 471-630 | 471-493 | SMP | DNA | Human | 4 |
| IV | 631-790 | 631-653 | SMP | Protein | Human | 5 |
| V | 791-897 | 791-814 | SMN | DNA | Rat | 6 |
| VI | 898-962 | 898-914 | SMN | Protein | Rat | 7 |
| VII | 963-1038 | 963-979 | SMN | DNA | Human | 8 |
| VIII | 1039-1114 | 1039-1055 | SMN | Protein | Human | 9 |
| IX | 1115-1163 | 1115-1120 | BMN | DNA | Rat | 10 |
| X | 1164-1178 | 1164-1166 | BMN | Protein | Rat | 11 |
| XI | 1179-1207 | 1179-1182 | BMN | DNA | Human | 12 |
| XII | 1208-1236 | 1208-1211 | BMN | Protein | Human | 13 |

| | | | | | | |
|---|---|---|---|---|---|---|
| * SMP - surrogate marker of neuropathic pain; SMN - surrogate marker of neurotrophic activity; BMN - biomarker of a neurotrophic agent. ** Here and in all tables, DNA denotes any nucleic acid, including, e.g, mRNA and DNA. | | | | | | |

**Table 1a.**

| **Group No.** | **SEQ ID NOs:** | **Preferred SEQ ID NOs:** | **Category*** | **Type** | **Species** | **Table No.** |
|---|---|---|---|---|---|---|
| XIII | 1252-1264 | 1252-1256 | BMN | DNA | Rat | 16 |
| XIV | 1265-1274 | 1265-1269 | BMN | Protein | Rat | 17 |
| XV | 1276-1287 | 1276-1280 | BMN | DNA | Human | 18 |
| XVI | 1288-1299 | 1288-1292 | BMN | Protein | Human | 19 |

Various embodiments of the invention are set forth in the following description or will be understood from the description.

### BRIEF DESCRIPTION OF THE FIGURES

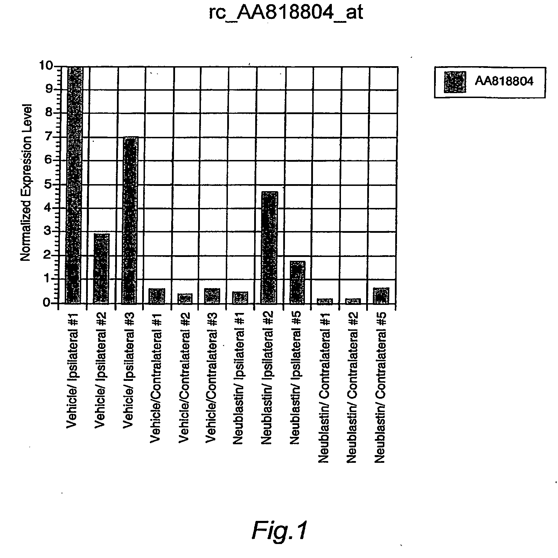

Figure 1 shows results of a TaqMan^{™} analysis of gene expression of rc_AA818804_at (SEQ ID NO:18 and SEQ ID NO:799) in the L4 dermatome of rats subjected to spinal nerve ligation injury (SNL) and treament with artemin. The gene is expressed at a low level before injury, at a higher level following injury, and at a near-normal level after injury and treatment with artemin.

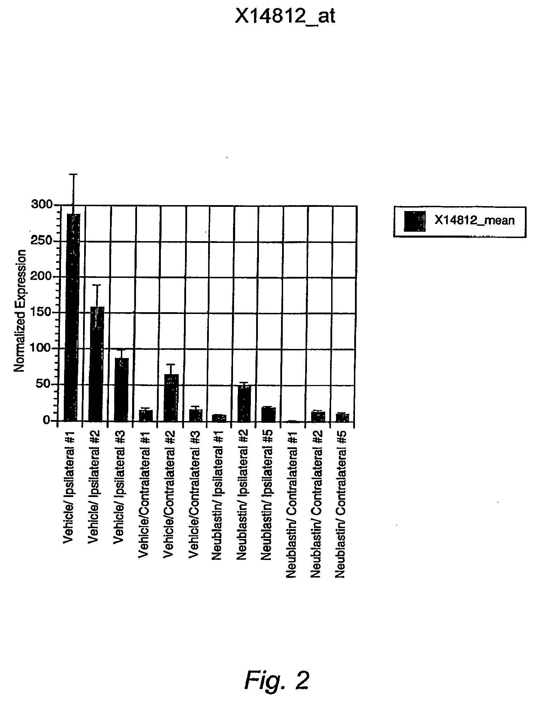

Figure 2 shows results of a TaqMan^{™} analysis of gene expression of X14812_at (SEQ ID NO:37 and SEQ ID NO:813) in the L4 dermatome of rats subjected to SNL and treament with artemin. The gene is expressed at a low level before injury, at a higher level following injury, and at a near-normal level after injury and treatment with artemin.

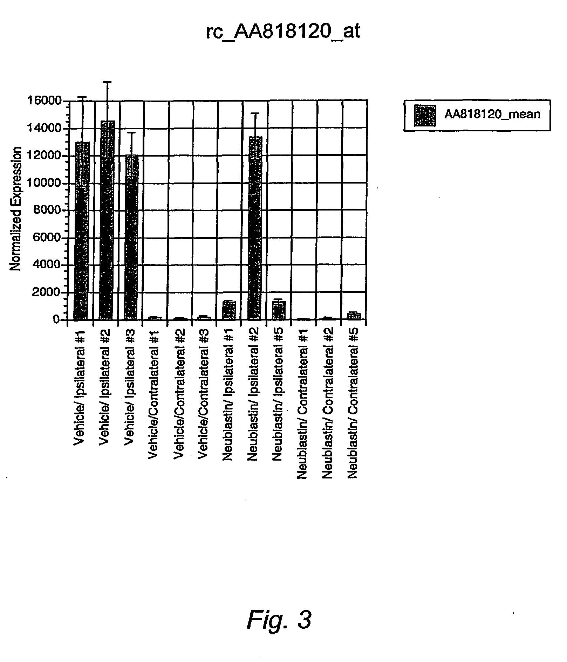

Figure 3 shows results of a TaqMan^{™} analysis of gene expression of rc_AA818120_at (SEQ ID NO:31 and SEQ ID NO:808) in the L4 dermatome of rats subjected to SNL and treament with artemin. The gene is expressed at a low level before injury, at a higher level following injury, and at a near-normal level after injury and treatment with artemin.

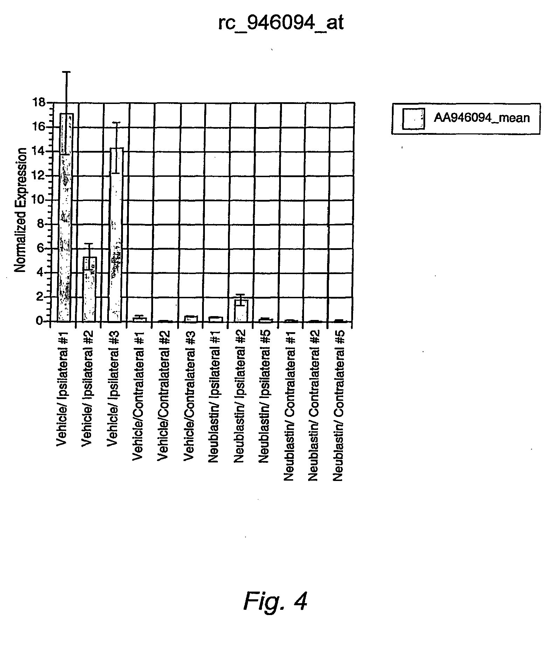

Figure 4 shows results of a TaqMan^{™} analysis of gene expression of rc_AA946094_at (SEQ ID NO:2 and SEQ ID NO:791) in the L4 dermatome of rats subjected to SNL and treament with artemin. The gene is expressed at a low level before injury, at a higher level following injury, and at a near-normal level after injury and treatment with artemin.

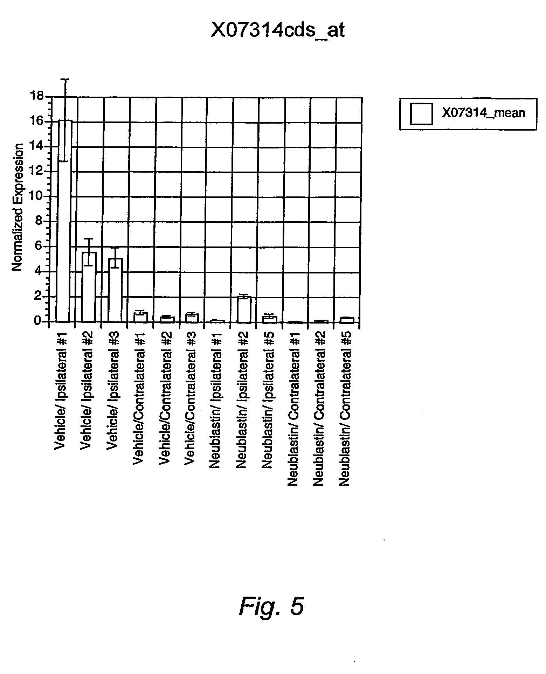

Figure 5 shows results of a TaqMan^{™} analysis of gene expression of X07314ods_at (SEQ ID NO:11 and SEQ ID NO:796) in the L4 dermatome of rats subjected to SNL and treament with artemin. The gene is expressed at a low level before injury, at a higher level following injury, and at a near-normal level after injury and treatment with artemin.

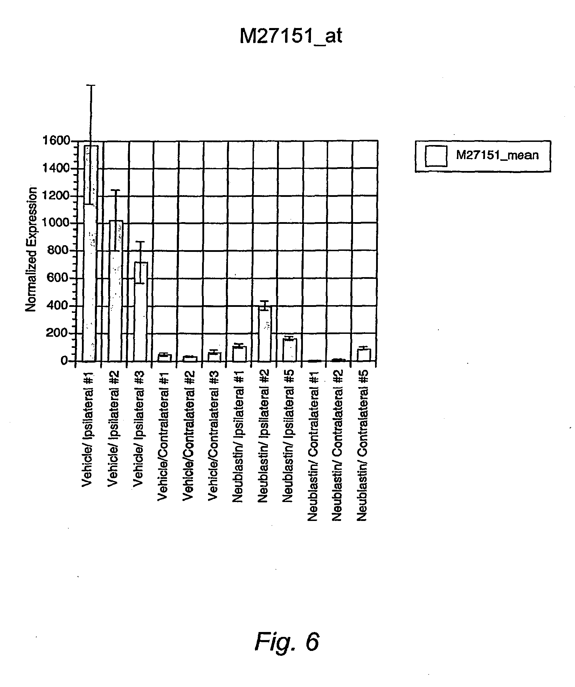

Figure 6 shows results of a TaqMan^{™} analysis of expression of gene M27151_at (SEQ ID NO:22 and SEQ ID NO:801) in the L4 dermatome of rats subjected to SNL and treament with artemin. The gene is expressed at a low level before injury, at a higher level following injury, and at a near-normal level after injury and treatment with artemin.

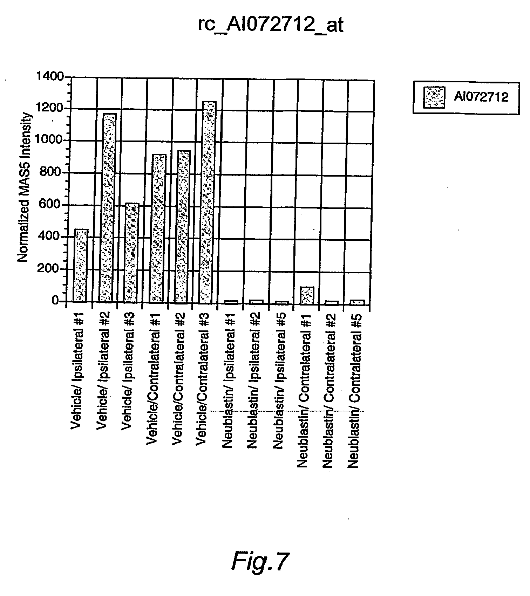

Figure 7 shows results of an Affymatrix analysis of expression of gene rc_Al072712_at (SEQ ID NO: 1118) in the L4 dermatome of rats subjected to SNL and treament with artemin. Regardsless of injury state, this gene is expressed at a relatively high level in the vehicle-treated samples, and at a much reduced level following treatment with artemin.

### DETAILED DESCRIPTION OF THE INVENTION

In the experiments leading to the present invention, rats were subjected to unilateral spinal nerve ligation (SNL) to induce unilateral neuropathic pain. Following SNL, some rats were systemically administered artemin, a neurotrophic factor shown to reduce neuropathic pain (Gardell et al. (2003) Nature Med., 9(11):1383-1389). The induced neuropathic pain was assessed using behavioral tests. Skin samples were then obtained bilaterally and tissue extracts were prepared. RNA from these tissue extracts was subjected to Affymetrix GeneChip^{™} expression analysis to determine gene expression profiles in various samples.

The heterogeneity of tissues usually makes it difficult to detect small changes in transcription in tissue samples, especially if the changes are restricted to small subpopulations of cells or are a result of indirect efFects. Despite this difficulty, the present invention is based, in part, on the di scovery and demonstration that detectable changes in gene expression in skin biopsy homogenates reflect the neuropathic pain status.

In particular, the methods of the invention may be used to identify genes whose expression levels correlate with neuropathic pain (surrogate markers of neuropathic pain). The invention may be also used to identify a subset of these genes whose expression levels are at least partially normalized by the artemin treatment (surrogate markers of neurotrophic activity). The invention may be used to identify an additional set of genes whose expression levels correlate with the presence of biologically active artemin regardless of the neuropathic pain status (biomarkers of a neurotrophic agent).

### Surrogate markers of neuropathic pain

The invention provides a method of identifying a surrogate marker of neuropathic pain in a mammal, comprising:
(a) obtaining a first skin biopsy sample under conditions of neuropathic pain;
(b) obtaining a second skin biopsy sample under conditions of substantially no neuropathic pain;
(c) preparing tissue extracts from the first and the second sam pies; and
(d) determining an amount of at least one nucleic acid or protein in the tissue extracts;
wherein a difference between the amount of the nucleic acid or the protein in the first sample and the amount of the same nucleic acid or protein in the second sample indicates that the nucleic acid or the protein is a surrogate marker of neuropathic pain. In some embodiments, the amount of the nucleic acid or the protein in the first sample will differ from the amount of the same nucleic acid or protein in the second sample by, for example, 2, 3, 4, 5, 8, 10, 20, 30, 40, 50, 80, 100-fold, or more. The difference (also referred to as "fold-change") indicates a correlation of the downregulation or upregulation of the relevant gene and neuropathic pain. The greater the fold-change in expression and/or the higher the degree of correlation with neuropathic pain, the more preferable the nucleic acid or protein is as a surrogate marker of neuropathic pain.

The first and the second samples can be obtained from the same mammal or from different mammals. For example, the first and second samples can be obtained from the same mammal from different regions of the skin, one region affected by neuropathic pain or peripheral neuropathy, and the other region not affected by pain or neuropathy. In another example, the first and second samples can be obtained from the same region of the skin in the same mammal but at different times. For example, a first sample can be collected prior to inducing neuropathic pain and the second sample is obtained following induction of neuropathic pain. In yet another example, the first sample can be collected from the region affected by neuropathic pain, and the second sample is obtained from the same region following treatment. Alternatively, the first and second samples can be obtained from different mammals and the amounts of a nucleic acid or protein are compared with reference to a common control using statistical analysis.

Illustrative methods of identifying a surrogate marker of neuropathic pain in rats are provided in the Examples. 308 rat nucleic acids (Table 2) were identified following these illustrative methods. Corresponding protein sequences and human orthologues were then identified using publicly available databases such as GenBank™. 162 rat protein sequences (Table 3), 160 human nucleic add sequences (Table 4), and 160 human protein sequences (Table 5) were identified in this manner.

The invention provides a method of evaluating the level of neuropathic pain in a mammal, comprising:
(a) obtaining a first skin biopsy sample under conditions of neuropathic pain;
(b) obtaining a second skin biopsy sample under conditions of substantially no neuropathic pain;
(c) preparing tissue extracts from the first and the second samples; and
(d) determining an amount of at least one nucleic acid or protein in the tissues, the nucleic acid or the protein being a surrogate marker of neuropathic pain;
wherein a difference between the amount of the nucleic acid or the protein in the first sample and the amount of the same nucleic acid or protein in the second sample indicates the level of neuropathic pain. In some embodiments, the amount of the nucleic acid or the protein in the first sample will differ from the amount of the same nucleic acid or protein in the second sample by, for example, 2, 3, 4, 5, 8, 10, 20, 30, 40, 50, 80, 100-fold, or more. The difference ("fold-change") in the expression levels of a relevant surrogate marker of neuropathic pain correlates with the level, or degree, or neuropathic pain. Generally, surrogate markers of neuropathic pain that exhibit greater fold-change values indicate a higher degree of neuropathic pain.

The first and the second samples can be obtained from the same mammal or from different mammals as described herein.

In some embodiments, the surrogate marker of neuropathic pain is a nucleic acid. In illustrative embodiments, the nucleic acid comprises a nonredundant subsequence of any one of the rat nucleotide sequences of SEQ ID NOs:1-308, preferably SEQ ID NOs:1-42. In other illustrative embodiments, a surrogate marker of neuropathic pain is a nucleic acid that comprises a nonredundant subsequence of any one of the human nucleotide sequences of SEQ ID NOs:471-630, preferably SEQ ID NOs:471-493.

In some embodiments, the surrogate marker of neuropathic pain is a protein. In illustrative embodiments, the protein comprises a nonredundant subsequence of any one of the rat protein sequences of SEQ ID NOs:309-470, preferably SEQ ID NOs:309-333. In other illustrative embodiments, a surrogate marker of neuropathic pain is a protein that comprises a nonredundant subsequence of any one of the human protein sequences of SEQ ID NOs:631-790, preferably SEQ ID NOs:631-653.

Conditions in which neuropathic pain may occur, and therefore may require assessment in the course of diagnosis or treatment, include but are not limited to: traumatic (including iatrogenic) nerve injury, ischemic neuropathy, nerve compression/entrapment, polyneuropathy (hereditary, metabolic, toxic, inflammatory; infectious, paraneoplastic, nutritional, in amyloidosis and vasculitis), plexus injury root compression, stump and phantom pain after amputation, herpes zoster/postherpetic neuralgia, trigeminal and glossopharyngeal neuralgia, cancer-related neuropathy (due to neural invasion of the tumor, surgical nerve damage, radiation-induced nerve damage, chemotherapy-induced neuropathy), stroke (infarct or hemorrhage), multiple sclerosis, spinal cord injury, syringomyelia/syringobulbia, epilepsy, and space-occupying lesions. Examples of specific disorders include diabetic neuropathy, sensory neuropathy of AIDS and antiretroviral toxic neuropathy, idiopathic small fiber neuropathy, leprosy, Fabry disease. Additionally, the method of assessing neuropathic pain may be used to assess induced neuropathic pain in experimental animals, e.g., SNL-induced neuropathic pain in rats as described in the Examples.

Assessment of pain with the methods of the invention may be conducted in the course of pharmacological and/or nonpharmacological treatments. Nonpharmacological treatments of neuropathic pain include transcutaneous electrical nerve stimulation, spinal cord stimulation, motor cortex stimulation, deep brain stimulation, decompression, neuroma removal, neurotomy, glycerol injection, radiofrequency nerve/root lesion, dorsal root entry zone lesion, and cordotomy.

### Surrogate markers of neurotrophic activity

A subset of surrogate markers of neuropathic pain is expected to be normalized as a result of a treatment with a compound or a composition that reduces neuropathic pain.

Accordingly, the invention provides a method of evaluating the effect of a compound or composition on the level of neuropathic pain in a mammal, comprising:
(a) administering the compound or composition to the mammal having neuropathic pain;
(b) obtaining at least one skin biopsy sample from the mammal;
(c) preparing a tissue extract from the skin biopsy sample; and
(d) determining an amount of at least one nucleic acid or protein in the tissue extract, the nucleic acid or the protein being a surrogate marker of neuropathic pain;
wherein a difference in the amount of the nucleic acid or protein determined in step (d) and the amount of the same nucleic acid or protein expressed in the absence of the compound or composition indicates the level of efficacy of the compound or composition on neuropathic pain.

The amount of a nucleic acid or protein expressed in the absence of the compound or composition can be determined by any suitable method. In one method, the amount of the nucleic acid or protein in the test sample is compared to the amount of the same nucleic acid or protein in another sample obtained in the absence of the compound or composition from the same mammal or from different mammals. The control sample may be collected before, during, or after the analysis. In another method, the amount of the nucleic acid or protein in the test sample is compared to that of one or more internal references. An internal reference is a nucleic acid or a protein whose expression levels under given conditions are known. Most typically, the reference is a gene that remains relatively constant under various conditions such as a housekeeping gene, e.g., actin or GAPDH.

In some embodiments, the amount determined in step (d) will differ from the amount of the same nucleic acid or protein expressed in the absence of the compound or composition by, for example, 2, 3, 4, 5, 8, 10, 20, 30, 40, 50, 80, 100-fold, or more. The "normalization" of the expression level of a relevant surrogate marker of neuropathic pain towards the baseline expression level as in normal conditions (substantially no neuropathic pain) indicates that the compound or composition reduces neuropathic pain. The difference in expression levels under conditions of neuropathic pain and upon "normalization" ("fold-change-back") indicates the level of neurotrophic activity of the compound or composition being evaluated. Generally, the greater fold-change-back values indicate that the compound or composition is expected to exhibit greater efficacy in treating neuropathic pain. Although greater fold-change-back values are preferred, it is also preferred that a fold-change-back value for a particular surrogate marker of neuropathic pain does not substantially exceed a corresponding fold-change value for the marker.

Illustrative methods of evaluating the effect of a compound or composition on the level of neuropathic pain in rats are provided in the Examples. 107 rat nucleic acids (Table 6) were identified following these methods. Corresponding protein sequences and human orthologues were then identified using publicly available databases such as GenBank^{™}. 65 rat protein sequences (Table 7), 76 human nucleic acid sequences (Table 8); and 76 human protein sequences (Table 9) were identified in this manner.

In some embodiments, the surrogate marker of neurotrophic activity is a nucleic acid. In illustrative embodiments, the nucleic acid comprises a nonredundant subsequence of any one of the rat nucleotide sequences of SEQ ID NOs:791-897, preferably SEQ ID NOs:791-814. In other illustrative embodiments, a surrogate marker of neurotrophic activity is a nucleic acid that comprises a nonredundant subsequence of any one of the human nucleotide sequences of SEQ ID NOs:963-1038, preferably SEQ ID NOs:963-979.

In some embodiments, the surrogate marker of neurotrophic activity is a protein. In illustrative embodiments, the protein comprises a nonredundant subsequence of any one of the rat protein sequences of SEQ ID NOs:898-962, preferably SEQ ID NOs:898-914. In other illustrative embodiments, the surrogate marker of neurotrophic activity is a protein that comprises a nonredundant subsequence of any one of the human protein sequences of SEQ ID NOs:1039-1114, preferably SEQ ID NOs:1039-1055.

In some embodiments, the compound or composition to be evaluated is or comprises a neurotrophic agent. "Neurotrophic agent" is a compound that has neurotrophic activity, i.e., it affects generation, survival, growth, or maintenance of normal physiological function of neurons. Neurotrophic activity can be evaluated/measured by one or more methods known in the art, for example:
(1) RET kinase receptor activation ELISA (KIRA) (Milbrandt et al. (1998) Neuron, 20:245; Sadick et al., 1996, Anal. Biochem., 1996. 235(2):207);
(2) choline acyteltransferease enzymatic assays (Leibrock et al.(1989) Nature, 341:149;
(3) ³H-dopamine uptake assay with dopaminergic neurons (Lev-Fen et al. (1993) Science, 260:1130; or
(4) rat pheochromocytome cell line PC12 assays (Emfors et al. (1991) Nature, 350:1756; Darling et al. (1984) Methods for preparation and assay of nerve growth factor", Cell Culture Methods for Molecular and Cellular Biology, vol. 4 (eds. Bames et al.), pp.79-83, Alan R. Liss, New York; Bradshaw (1978) Ann Rev. Biochem, 47:191).

In illustrative embodiments, the neurotrophic agent being evaluated is artemin. Other examples of neurotrophic agents include neurotrophic factors such as other members of the GDNF family (e.g., GDNF, neurturin, persephin), nerve growth factor (NGF), brain-derived neurotrophic factor (BDNF), neutrotrophin-3 (NT-3), leukocyte migration inhibitory factor (LIF), interleukin 6 (IL6), basic fibroblast growth factor (bFGF), midkine, neutrotrophin-4 (NT4), ciliary neurotrophic factor (CNTF), pleiotrophin, epidermal growth factor (EGF), hepatocyte growth factor (HGF), vascular endothelial growth factor (VEGF), and insulin-like growth factor type 1 (IGF-1). Yet other examples of neurotrophic agents include agonists and antagonists of these neurotrophic factors or their respective receptors. Examples of agonist and/or antagonists include antibodies against a neurotrophic factor or their receptors and soluble forms of the receptors such as GFR-α (receptor for neurturin); RETα4 (receptor for persephin); GFRα3 (receptor for artemin), TrkA (receptor for NGF), TrkB (receptor for BDNF), TrkC (receptor for NT-3), gp130/LIFRβ (receptor for LIF), and gp130 (receptor for IL6).

In some embodiments, the compound or composition to be evaluated is a drug or drug candidates for treating neuropathies and include neurotrophic agents as described herein. Examples of drugs that are currently used for the treatment of neuropathic pain, and therefore may be evaluated for neurotrophic activity, include antidepressants (amitriptyline, maprotiline, selective serotonin reuptake inhibitors), antiepileptics (gabapentin, carbamazepine, clonazepam, lamotrigine, topiramate, phenytoin), local anesthetics, mexiletine, baclofen, clonidine, ketamine, dextrorphan, tramadol, guanethidine, and opioids (morphine, methadone, ketobemidone, fentanyl).

### Biomarkers of neurotrophic agents

The invention provides a method of identifying a biomarker of biological activity of a "neurotrophic agent" (as described herein). The method comprises:
(a) administering the agent to a mammal;
(b) obtaining at least one skin biopsy sample from the mammal;
(c) preparing a tissue extract from the skin biopsy sample; and
(d) determining an amount of at least one nucleic acid or protein in the tissue extract;
wherein a difference in the amount of the nucleic acid or protein determined in step (d) and the amount of the same nucleic acid or protein expressed in the absence of the agent indicates that the nucleic acid or the protein is a biomarker of in vivo biological activity of the agent. In some embodiments, the amount determined in step (d) will differ from the amount of the same nucleic acid or protein expressed in the absence of the agent by, for example, 2, 3, 4, 5, 8, 10, 20, 30, 40, 50, 80, 100-fold, or more. The difference in the levels of expression that is attributed to the presence of biologically active neurotrophic agent is termed "blomarker-fold-change." The greater the biomarker-fold-change value is, the more preferable the nucleic acid or protein is as a biomarker of biological activity of a neurotrophic agent. Some biomarkers (e.g., SEQ ID N0:1120 and SEQ ID N0:1126) may also represent surrogate markers of pain, i.e., they correlate with both neuropathic pain and the presence of a biologically active neurotrophic agent. Additionally, some of these biomarkers (e.g., SEQ ID N0:1120 and SEQ ID NO:1126) may also serve as surrogate markers of neurotrophic activity.

The amount of the same nucleic acid or protein expressed in the absence of the compound or composition can be determined by any suitable method. The skin biopsy sample(s) can be obtained from the same mammal or from different mammals.

Illustrative methods of identifying a biomarker of biological activity of a "neurotrophic agent" in rats are provided in the Examples below. 49 rat nucleic acids (Table 10) were identified following these methods. 13 additional rat nucleic acides (Table 16) were identified following Example 2. Corresponding protein sequences and human orthologues were then identified using publicly available databases such as GenBank^{™}. 15 rat protein sequences (Table 11), 29 human nucleic acid sequences (Table 12), and 29 human protein sequences (Table 13) were identified in this manner. Additionaly, 11 rat protein sequences (Table 17), 12 human nucleic acid sequences (Table 18), and 12 human protein sequences (Table 19) were identified as corresponding to the sequences in Table 16.

The invention provides a method of evaluating biological activity of a neurotrophic agent, comprising:
(a) administering the agent to a mammal;
(b) obtaining at least one skin biopsy sample from the mammal;
(c) preparing a tissue extract from the skin biopsy sample; and
(d) determining an amount of at least one nucleic acid or protein in the tissue extract; the nucleic acid or protein being a biomarker of the biological activity of the neurotrophic agent;
wherein a difference in the amount of the nucleic acid or protein determined in step (d) and the amount of the same nucleic acid or protein expressed in the absence of the agent indicates that the agent is biologically active. In some embodiments, the amount determined in step (d) will differ from the amount of the same nucleic acid or protein expressed in the absence of the agent by, for example, 2, 3, 4, 5, 8, 10, 20, 30, 40, 50, 80, 100-fold, or more.

The amount of the same nucleic acid or protein expressed in the absence of the compound or composition can be determined by any suitable method. The skin biopsy sample(s) can be obtained from the same mammal or from different mammals.

In illustrative embodiments, the neurotrophic agent being evaluated is artemin, a member of the GDNF family.

In some embodiments, the biomarker of biological activity of a neurotrophic agent is a nucleic acid. In illustrative embodiments (see Example 1), the nucleic acid comprises a nonredundant subsequence of any one of the rat nucleotide sequences of SEQ ID NOs:1115-1163, preferably SEQ ID NOs:1115-1120. In other illustrative embodiments, a biomarker of biological activity of a neurotrophic agent is a nucleic acid that comprises a nonredundant subsequence of any one of the human nucleotide sequences of SEQ ID NOs:1179-1207, preferably SEQ ID NOs:1179-1182.

In yet other illustrative embodiments (see Example 2), the nucleic acid comprises a nonredundant subsequence of any one of the rat nucleotide sequences of SEQ ID NOs:1252-1264, preferably SEQ ID NOs:1252-1256. In other illustrative embodiments, a biomarker of biological activity of a neurotrophic agent is a nucleic acid that comprises a nonredundant subsequence of any one of the human nucleotide sequences of SEQ ID NOs:1276-1287, preferably SEQ ID NOs:1276-1280.

In some embodiments, the biomarker of biological activity of a neurotrophic agent is a protein. In illustrative embodiments (related to Example 1), the protein comprises a nonredundant subsequence of any one of the rat protein sequences of SEQ ID NOs: 1164-1178. preferably SEQ ID NOs:1164-1166. In other illustrative embodiments, a biomarker of biological activity of a neurotrophic agent is a protein that comprises a nonredundant subsequence of any one of the human protein sequences of SEQ ID NOs:1208-1236, preferably SEQ ID NOs:1208-1211.

In yet other illustrative embodiments (related to Example 2), the protein comprises a nonredundant subsequence of any one of the rat protein sequences of SEQ ID NOs:1265-1274, preferably SEQ ID NOs:1265-1269. In other illustrative embodiments, a biomarker of biological activity of a neurotrophic agent is a protein that comprises a nonredundant subsequence of any one of the human protein sequences of SEQ ID NOs:1288-1299, preferably SEQ ID NOs:1288-1292.

### General Methods

Various methods for obtaining skin biopsies are available. The least invasive is removal of the epidermis by placing a suction capsule with over the skin for 30-90 min to develop the blister. The epidermis separates cleanly at the dermal-epidermal junction (Kennedy et al. (1999) Muscle Nerve, 98:323-329; United States Patent No. 6,071,247). This approach is painless and occurs without bleeding because all of the blood vessels terminate beneath the epidermis in the dermal papillae. For these reasons it may be particularly safe on, for example, the feet of diabetic patients. Another approach is simple punch biopsy of the skin. This procedure is also well tolerated. If the biopsy diameter is restricted to 3 mm or less no suture is needed. The biopsy site heals by granulation and leaves a small circular scar that gradually resolves.

Expression levels, at the RNA or at the protein level, can be determined using conventional methods. Expression levels are usually scaled and/or normalized per total amount of RNA or protein in the sample and/or a control, which is typically a housekeeping gene such actin or GAPDH). RNA levels may be determined by, e.g., quantitative PCR (e.g., TaqMan^{™} PCR or RT-PCR), Northern blotting, or any other method for determining RNA levels, e.g., as described in Sambrook et al. (eds.) Cloning: A Laboratory Manual, 2nd ed., Cold Spring Harbor Laboratory Press, 1989, or Lodie et al. (2002) Tissue Eng. , 8(5):739-751), or as described in the Examples. Protein levels may be determined, .e.g., by using Western blotting, ELISA, enzymatic activity assays, or any other method for determining protein levels, e.g., as described in Current Protocols in Molecular Biology (Ausubel et al. (eds.) New York: John Wiley and Sons, 1998).

One or more markers of the same or different type can be used in the in the methods of the invention. For example, 1, 2, 3, 4, 5 or more nucleic acids and/or 1, 2, 3, 4, 5 or more proteins can be used for a read-out for (a) neuropathic pain, (b) effect of a compound or composition on the level of neuropathic pain, and/or (c) evaluating biological activity of a neurotrophic agent.

While representative procedures shown in the Examples are performed using rodents, a skilled artisan will recognize that such procedures can be successfully performed in other mammal and within parameters clinically feasible in human subjects. For example, skin biopsies can be obtained from human patients having neuropathic pain and then subjected to a similar analysis as described herein. For human samples, commercially or custom-made human gene arrays can be used (e.g., Affymatrix^{™} Human Genome sets U133, U133A, and U95).

The term "nonredundant subsequence," as used herein, refers to a subsequence which is unique to the sequence in which it occurs. In some embodiments, a nonredunant subsequence is at least, for example, 10, 15, 20, 30, 40, 50, 70, 100, 200, 300, 400, 500, 1000, or 1500 nucleotides long.

All or some of the following sequences and their nonredundant subsequences can be excluded from certain embodiments: (a) rat DNA SMPs as set out in SEQ ID NOs: 8, 15, 100, 171, 199, 244; (b) rat protein SMPs as set out in SEQ ID NOs: 315, 318, 408, 420; (c) human DNA SMPs as set out in SEQ ID NOs: 476, 478, 568, 578; (d) human protein SMP sas set out in SEQ ID NOs: 636, 638, 728, 738; (e) rat DNA SMNs as set out in SEQ ID NOs: 798, 834; (f) rat protein SMN set out in SEQ ID NO:903; (g) human DNA SMN set out in SEQ ID NO:967; (h) human protein SMN as set out in SEQ ID N0:1043; and (i) sequences disclosed U.S. Patent Application Publication No. US2003/0216341.

**Table 2. Rat DNA SMPs**

| **SEQ ID NO:** | **AffyID^{™}** | **Accession Number** |
|---|---|---|
| 1 | rc_AI639444_at | NM_057191* |
| 2 | rc_AA946094_at | NM_021588 |
| 3 | rc_AA891522_f_at | NM_017240* |
| 4 | rc_AA799471_at | AA799471 |
| 5 | rc_Al172339_at | NM_175844 |
| 6 | U31816_s_at | U31816 |
| 7 | M24393_at | NM_017115* |
| 8 | M98819mRNA_s_at | M98819 |
| 9 | rc_AI010701_at | AI010701 |
| 10 | rc_AI010736_at | A1010736 |
| 11 | XO7314cds_at | X07314* |
| 12 | rc_AI639444_g_at | NM_057191* |
| 13 | rc_AA799396_at | AA799396 |
| 14 | rc_AI169831_at | AI169831 |
| 15 | rc_AI012182_s_at | NM_033234 |
| 16 | LO4684_at | L04684 |
| 17 | rc_AI171653_at | AI171653 |
| 18 | rc_AA818804_at | AA818804 |
| 19 | rc_Al044544_at | Al044544 |
| 20 | M12098_s_at | NM_012604* |
| 21 | rc_Al073178_at | Al073178 |
| 22 | M27151_at | NM_013172* |
| 23 | rc_Al227690_at | Al227690 |
| 24 | rc_Al175100_at | Al175100 |
| 25 | AF077338_at | NM_031813* |
| 26 | X74832cds_at | NM_024485* |
| 27 | rc_AI105049_at | AI105049 |
| 28 | rc_AA866452_s_at | AA866452* |
| 29 | AA108284_at | NM_019292* |
| 30 | rc_AI104913_at | NM_013044* |
| 31 | rc_AA818120_at | AA818120 |
| 32 | X59864mRNA_at | X59864 |
| 33 | AF039832_g_at | NM_019334 |
| 34 | X15939_i_at | NM_017240* |
| 35 | rc_AA851497_f_at | AA851497 |
| 36 | rc_AA818845_at | AA818845 |
| 37 | X14812_at | NM_012606* |
| 38 | rc_AA800206_at | AA800206* |
| 39 | X15939_r_at | NM_017240* |
| 40 | rc_AA901245_at | AA901245 |
| 41 | X59864mRNA_g_at | X59864 |
| 42 | rc_AA924417_f_at | NM_053395* |
| 43 | rc_AI170763_at | AI170763 |
| 44 | rc_AI170764_at | AI170764 |
| 45 | rc_AA818947_at | AA818947 |
| 46 | X81193_at | NM_057144* |
| 47 | rc_AI170760_at | AI170760 |
| 48 | X80130cds_i_at | X80130 |
| 49 | rc_AA818952_at | AA818952 |
| 50 | rc_A819140_at | NM_019292* |
| 51 | rc_Al170696_at | NM_133583 |
| 52 | rc_Al170687_at | Al170687 |
| 53 | rc_AA998888_f_at | AA998888* |
| 54 | rc_AA819891_at | AA819891 |
| 55 | rc_AA998685_f_at | AA998685* |
| 56 | rc_AA998374_f_at | AA998374 |
| 57 | rc_AA849917_at | AA849917 |
| 58 | rc_AA819868_at | AA819868 |
| 59 | rc_AA849501_s_at | AA849501 * |
| 60 | rc_AA819699_at | AA819699 |
| 61 | rc_AA892801_at | NM_017245 |
| 62 | rc_AA875288_at | AA875288 |
| 63 | rc_AA891037_at | AA891037 |
| 64 | rc_AA891903_at | AA891903 |
| 65 | rc_AA891938_at | AA891938 |
| 66 | rc_AA892287_at | AA892287 |
| 67 | rc_AA892313_at | AA892313 |
| 68 | rc_AI172259_at | AI172259 |
| 69 | rc_AA892468_g_at | NM_138836 |
| 70 | rc_AA859829_g_at | AA859829 |
| 71 | rc_AA892860_g_at | AA892860 |
| 72 | rc_AA892999_at | AA892999 |
| 73 | rc_AA893195_at | AA893195 |
| 74 | rc_AA893199_at | AA893199 |
| 75 | rc_AA893307_at | AA893307 |
| 76 | rc_AA894101_g_at | AA894101 |
| 77 | rc_AA892468_at | NM_138836 |
| 78 | rc_Al233870_at | Al233870 |
| 79 | X96437mRNA_g_at | X96437 |
| 80 | Y09453cds_at | NM_019255 |
| 81 | Z78279_at | Z78279 |
| 82 | rc_Al172054_at | Al172054 |
| 83 | rc_Al172150_at | Al172150 |
| 84 | rc_Al172171_at | Al172171 |
| 85 | rc_AA875206_at | NM_053747 |
| 86 | rc_AI172189_at | A1172189 |
| 87 | rc_AA859931_g_at | AA859931 |
| 88 | rc_AI233915_at | AI233915 |
| 89 | rc_AI236229_at | AI236229 |
| 90 | rc_AA849974_at | AA849974 |
| 91 | rc_AA858869_at | AA858869 |
| 92 | rc_AA858921_at | AA858921 |
| 93 | rc_AA859335_at | NM_017147* |
| 94 | X90475cds_at | X90475* |
| 95 | rc_AI172183_at | AI172183 |
| 96 | K03467_s_at | NM_012604* |
| 97 | H32169_at | H32169 |
| 98 | H32451_at | H32451 |
| 99 | J00692_at | J00692* |
| 100 | J01435cds#8_s_at | J01435 |
| 101 | J01436cds_s_at | J01436 |
| 102 | J04993_at | NM_017184* |
| 103 | L11694_at | NM_017033 |
| 104 | K02423cds_s_at | NM_020104* |
| 105 | D38056_at | NM_053599 |
| 106 | L00088exp_cds#2_at | NM_020104* |
| 107 | L00382cds_at | L00382* |
| 108 | L01702_at | NM_012763 |
| 109 | L01793_at | NM_031043 |
| 110 | L01793_g_at | NM_031043 |
| 111 | rc_AA799773_g_at | AA799773 |
| 112 | K02111_at | K02111* |
| 113 | AF052540_s_at | NM_017117 |
| 114 | AA942808_at | AA942808 |
| 115 | AB000216_at | NM_134403 |
| 116 | AB009999_g_at | NM_031242 |
| 117 | AF002281_at | NM_053650 |
| 118 | AF008439_at | NM_013173 |
| 119 | AF013144_at | NM_133578 |
| 120 | E12625cds_at | NM_080886 |
| 121 | AF037072_at | NM_019292* |
| 122 | D64046_at | NM_022185 |
| 123 | AF061726_s_at | NM_017117* |
| 124 | AF077338_g__pt | NM_031813* |
| 125 | AF080507_at | AF080507 |
| 126 | AF086624_s_at | NM_022602 |
| 127 | AF093536_at | NM_031810 |
| 128 | D37920_at | NM_017136 |
| 129 | L13606_at | L13606* |
| 130 | AF030089UTR#1_at | NM_021584 |
| 131 | rc_AA800245_at | AA800245* |
| 132 | M83298_g_at | NM_053999 |
| 133 | M83676_at | M83676 |
| 134 | M84176_at | NM_176079* |
| 135 | M86621_at | NM_012919 |
| 136 | M89945mRNA_g_at | M89945 |
| 137 | rc_AA799571_at | AA799571 |
| 138 | L08505_at | NM_019226 |
| 139 | rc_AA800221_at | NM_053395* |
| 140 | M57263_at | NM_031659 |
| 141 | rc_AA800637_at | AA800637 |
| 142 | rc_AA817802_at | AA817802 |
| 143 | rc_AA817929_at | AA817929 |
| 144 | rc_AA817969_at | AA817969 |
| 145 | rc_AA817975_at | NM_031355 |
| 146 | rc_AA818745_at | AA818745 |
| 147 | rc_AA799773_at | AA799773 |
| 148 | M21759mRNA_at | M21759 |
| 149 | L24897_s_at | L24897* |
| 150 | L27124_s_at | NM_012993 |
| 151 | L28818cds_at | NM_022195 |
| 152 | M10140_at | NM_012530* |
| 153 | M13100cds#2_s_at | M13100 |
| 154 | M16112_at | NM_021739 |
| 155 | M63122_at | NM_013091 |
| 156 | M18330_at | NM_133307 |
| 157 | M62752_at | NM_012660 |
| 158 | M23995_g_at | NM_017272 |
| 159 | M27434_s_at | NM_147214 |
| 160 | M32397_at | NM_020072 |
| 161 | M37941mRNA_s_at | NM_138876 |
| 162 | M37942 exon#2-3_s_at | M37942 |
| 163 | M55534mRNA_s_at | NM_012935 |
| 164 | rc_AA818807_at | AA818807 |
| 165 | M16112_g_at | NM_021739 |
| 166 | rc_AI232024_f_at | NM_017239* |
| 167 | rc_AI180281_at | NM_175843 |
| 168 | rc_AI180442_at | NM_031840 |
| 169 | rc_AI227677_at | AI227677 |
| 170 | rc_AI230247_s_at | NM_019192 |
| 171 | rc_AI230319_at | NM_171992 |
| 172 | rc_AI230596_at | A1230596 |
| 173 | rc_AI639187_at | AI639187 |
| 174 | rc_AI231572_at | A1231572 |
| 175 | rc_AI178893_at | AI178893 |
| 176 | rc_Al236301_at | AI236301 |
| 177 | rc_AI237371_at | NM_031812 |
| 178 | rc_AI237700_at | AI237700 |
| 179 | rc_AI638960_at | AI638960 |
| 180 | rc_AI638986_s_at | AI638986 |
| 181 | rc_AI171376_at | NM_053395* |
| 182 | rc_AI231279_at | AI231279 |
| 183 | rc_AI175328_at | AI175328 |
| 184 | rc_AA945861_at | AA945861 |
| 185 | rc_AI171774_at | Al171774* |
| 186 | rc_AI172006_at | NM_021666 |
| 187 | rc_AI172423_at | NM_181368 |
| 188 | rc_AI172597_at | Al172597 |
| 189 | rc_AI175011_at | AI175011 |
| 190 | rc_AI179243_at | NM_145775 |
| 191 | rc_AI175258_at | Al175258 |
| 192 | rc_AI178921_s_at | NM_013159 |
| 193 | rc_AI175348_at | AI175348 |
| 194 | rc_AI175507_at | AI175507 |
| 195 | rc_AI175539_at | NM_022499* |
| 196 | rc_AI175935_at | NM_173101* |
| 197 | rc_AI176584_at | NM_012817 |
| 198 | rc_AI178559_at | NM_012923 |
| 199 | rc_AI639233_s_at | AI639233 |
| 200 | rc_AI175045_at | AI175045 |
| 201 | X70871_at | NM_012923 |
| 202 | X52311_at | NM_054006 |
| 203 | X53504cds_at | X53504 |
| 204 | X53504cds_g_at | X53504 |
| 205 | X56133_at | X56133* |
| 206 | X60351cds_s_at | NM_012935 |
| 207 | X64401cds_s_at | NM_173144 |
| 208 | rc_AI639178_at | AI639178 |
| 209 | X70369_s_at | X70369 |
| 210 | X04267_at | NM_012604* |
| 211 | X74835cds_at | NM_019298 |
| 212 | X76489cds_g_at | X76489 |
| 213 | X78848cds_f_at | NM_031509 |
| 214 | X80130cds_f_at | X80130* |
| 215 | Z78279_g_at | Z78279 |
| 216 | Z83869cds_at | NM_021699 |
| 217 | X64827cds_s_at | NM_012786* |
| 218 | U20195_s_at | NM_017033 |
| 219 | rc_Al639324_at | Al639324 |
| 220 | rc_Al639410_i_at | Al639410 |
| 221 | rc_AI639410_s_at | AI639410 |
| 222 | rc_AI639465_f_at | NM_080903* |
| 223 | rc_AI639532_at | AI839532 |
| 224 | rc_H33725_at | NM_138531 |
| 225 | X15939_f_at | NM_017240* |
| 226 | S74265_s_at | NM_013066 |
| 227 | X12554cds_s_at | NM_012812 |
| 228 | U25651_at | NM_031715* |
| 229 | U30938_at | NM_013066 |
| 230 | U40836mRNA_s_at | NM_012786 |
| 231 | U50736_s_at | NM_013220 |
| 232 | U84727 at | NM_022398 |
| 233 | U96130_at | NM_031043 |
| 234 | rc_AI171372_at | AI171372 |
| 235 | S49760_at | NM_080787 |
| 236 | rc_AI029057_at | AI029057 |
| 237 | rc_AI010583_at | NM_133424 |
| 238 | rc_AI010605_at | AI010605* |
| 239 | rc_AI010742_at | Al010742 |
| 240 | rc_AI011563_s_at | AI011563 |
| 241 | rc_AI011709_at | AI011709 |
| 242 | rc_AI011855_at | AI011855 |
| 243 | rc_AI070208_at | Al070208 |
| 244 | rc_AI014135_g_at | Al014135 |
| 245 | rc_AA996612_at | AA996612 |
| 246 | rc_AI029152_at | Al029152 |
| 247 | rc_AI030091_at | Al030091 |
| 248 | rc_Al043640_at | Al043640 |
| 249 | rc_Al044292_s_at | Al044292 |
| 250 | rc_Al045097_at | Al045097 |
| 251 | rc_AI171535_s_at | AI171535 |
| 252 | rc_AI014132_at | AI014132 |
| 253 | rc_AA946469_at | AA946469 |
| 254 | rc_AA924500_at | AA924500 |
| 255 | rc_AA925122_at | AA925122 |
| 256 | rc_AA925342_at | AA925342 |
| 257 | rc_AA925664_at | AA925664 |
| 258 | rc_AA944401_at | AA944401 |
| 259 | rc_AA944560_at | NM_153469 |
| 260 | rc_AI010562_at | AI010562* |
| 261 | rc_AA946457_at | AA946457 |
| 262 | rc_AA997341_at | NM_053326 |
| 263 | rc_AA955927_at | AA955927 |
| 264 | rc_AA957123_at | AA957123 |
| 265 | rc_AA963167_at | AA963167 |
| 266 | rc_AA963627_at | AA963627 |
| 267 | rc_AA963742_at | AA963742 |
| 268 | rc_AA964584_at | AA964584 |
| 269 | rc_AI070399_at | AI070399 |
| 270 | rc_AA946108_at | NM_173306 |
| 271 | rc_AI168935_at | AI168935 |
| 272 | rc_AI104924_f_at | NM_017239* |
| 273 | rc_AI059955_s_at | NM_053959 |
| 274 | rc_AI112050_at | AI112050 |
| 275 | rc_AI112084_at | AI112084 |
| 276 | rc_AI113309_at | AI113309 |
| 277 | rc_AI136540_at | AI136540* |
| 278 | rc_AA924428_at | AA924428 |
| 279 | rc_AI145367_at | NM_053874 |
| 280 | rc_AI104864_g_at | A1104864 |
| 281 | rc_AI169265_at | AI169265 |
| 282 | rc_AI170777_at | NM_024398 |
| 283 | rc_AI170777_g_at | NM_024398 |
| 284 | rc_AI170793_at | AI170793 |
| 285 | rc_AI170894_at | A1170894 |
| 286 | rc_Al170985_at | NM_020104* |
| 287 | rc_Al171098_at | Al171098* |
| 288 | rc_Al137958_at | Al137958 |
| 289 | rc_Al103376_at | Al103376 |
| 290 | rc_Al071299_at | NM_031135 |
| 291 | rc_Al071328_at | Al071328 |
| 292 | rc_Al071769_at | Al071769 |
| 293 | rc_Al072166_at | Al072166 |
| 294 | rc_Al101481_at | Al101481 |
| 295 | rc_Al111401_s_at | Al111401 |
| 296 | rc_AI102103_g_at | NM_031083 |
| 297 | rc_AI103473_at | NM_021865 |
| 298 | rc_AI103507_at | AI103507 |
| 299 | rc_AI103920_f_at | NM_017239* |
| 300 | rc_AI104035_s_at | AI104035 |
| 301 | rc_Al104326_at | Al104326 |
| 302 | rc_Al104349_at | Al104349 |
| 303 | rc_Al104354_at | Al104354 |
| 304 | rc_Al102057_at | Al102057 |
| 305 | rc_Al104567_g_at | Al104567* |
| 306 | rc_AA892861_at | AA892861 |
| 307 | rc_Al179358_at | Al179358 |
| 308 | AA799397_at | AA799397 |

**Table 3 Rat Protein SMPs.**

| **SEQ ID NO:** | **AffyID^{™}** | **Accession Number** | **Table 2 NO: SEQ ID NO:** |
|---|---|---|---|
| 309 | rc_AI639444_at | Q9ER30* | 1 |
| 310 | rc_AA946094_at | Q9QZ76 | 2 |
| 311 | rc_AA891522_f_at | P02564* | 3 |
| 312 | rc_AI172339_at | Q8K4K7 | 5 |
| 313 | U31816_s_at | Q02485 | 6 |
| 314 | M24393_at | P20428* | 7 |
| 315 | M98819 mRNA_s_at | NP_620231 | 8 |
| 316 | X07314cds_at | P08733* | 11 |
| 317 | rc_AI639444_g_at | Q9ER30* | 12 |
| 318 | rc_AI012182_s_at | P02091 | 15 |
| 319 | L04684_at | Q02485 | 16 |
| 320 | M12098_s_at | Q9QZV8* | 20 |
| 321 | M27151_at | P19335* | 22 |
| 322 | AF077338_at | 088599* | 25 |
| 323 | X74832cds_at | P25108* | 26 |
| 324 | rc_AA866452_s_at | P03996* | 28 |
| 325 | AA108284_at | P14141* | 29 |
| 326 | rc_Al104913_at | P70567* | 30 |
| 327 | X59864mRNA_at | Q03668** | 32 |
| 328 | AF039832_g_at | Q9R0W1 | 33 |
| 329 | X15939_i_at | P02564* | 34 |
| 330 | X14812_at | P16409* | 37 |
| 331 | X15939_r_at | P02564* | 39 |
| 332 | X59864mRNA_g_at | Q03668** | 41 |
| 333 | rc_AA924417_f_at | Q925F0* | 42 |
| 334 | X81193_at | P50463* | 46 |
| 335 | X80130cds_i_at | P04270 | 48 |
| 336 | rc_AA819140_at | P14141* | 50 |
| 337 | rc_AI170696_at | Q8VBU2 | 51 |
| 338 | rc_AA819891_at | Q9R272 | 54 |
| 339 | rc_AA849501_s_at | Q63518* | 59 |
| 340 | rc_AA892801 at | P05197 | 61 |
| 341 | rc_AA892468_g_at | Q9ES87 | 69 |
| 342 | rc_AA892468_at | Q9ES87 | 77 |
| 343 | Y09453cds_at | P97707 | 80 |
| 344 | Z78279_at | Q63079 | 81 |
| 345 | rc_AA875206_at | Q9JJP9 | 85 |
| 346 | rc_AA858869_at | AAO34127 | 91 |
| 347 | rc_AA859335_at | P45592* | 93 |
| 348 | X90475cds_at | Q63518* | 94 |
| 349 | K03467_s_at | Q9QZV8* | 96 |
| 350 | J00692_at | P02568* | 99 |
| 351 | J01436cds_s_at | AAA99907 | 101 |
| 352 | J04993_at | P13413* | 102 |
| 353 | L11694_at | P38652 | 103 |
| 354 | K02423cds_s_at | NP_064489* | 104 |
| 355 | D38056_at | P97553 | 105 |
| 356 | L00088exp_cds# | NP_064489* | 106 |
| 357 | L00382cds_at | AAA42289* | 107 |
| 358 | L01702_at | Q03348 | 108 |
| 359 | L01793_at | 008730 | 109 |
| 360 | L01793_g_at | 008730 | 110 |
| 361 | K02111_at | P04462* | 112 |
| 362 | AF052540_s_at | P16259 | 113 |
| 363 | AB000216_at | 008764 | 115 |
| 364 | AB009999_g_at | 035052 | 116 |
| 365 | AF002281_at | 070208 | 117 |
| 366 | AF008439_at | 054902 | 118 |
| 367 | AF013144_at | 054838 | 119 |
| 368 | E12625cds_at | 035532 | 120 |
| 369 | AF037072_at | P14141* | 121 |
| 370 | D64046_at | Q63788 | 122 |
| 371 | AF061726_s_at | P16259* | 123 |
| 372 | AF077338_g_at | O88599* | 124 |
| 373 | AF086624_s_at | 070444 | 126 |
| 374 | AF093536_at | 089117 | 127 |
| 375 | D37920_at | P52020 | 128 |
| 376 | L13606_at | Q07443* | 129 |
| 377 | AF030089UTR#1_at | Q9WVP7 | 130 |
| 378 | M83298_g_at | P36876 | 132 |
| 379 | M83676_at | P35284 | 133 |
| 380 | M84176_at | NP_788268* | 134 |
| 381 | M86621_at | P54290 | 135 |
| 382 | M89945mRNA_g_at | NP_114028 | 136 |
| 383 | L08505_at | P38650 | 138 |
| 384 | rc_AA800221_at | Q925F0* | 139 |
| 385 | M57263_at | P23606 | 140 |
| 386 | rc_AA817975_at | Q9R1Z0 | 145 |
| 387 | M21759mRNA_at | Q99053 | 148 |
| 388 | L24897_s_at | Q63350* | 149 |
| 389 | L27124_s_at | 25499 | 150 |
| 390 | L28818cds_at | NP_071531 | 151 |
| 391 | M10140_at | P00564* | 152 |
| 392 | M16112_at | Q63094 | 154 |
| 393 | M63122_at | P22934 | 155 |
| 394 | M18330_at | 170538 | 156 |
| 395 | M62752_at | P27706 | 157 |
| 396 | M23995_g_at | P13601 | 158 |
| 397 | M27434_s_at | P02761 | 159 |
| 398 | M32397_at | P20646 | 160 |
| 399 | M37941mRNA_s_at | P10759 | 161 |
| 400 | M37942exn#2-3 | NP_620231 | 162 |
| 401 | M55534mRNA_s_at | P23928 | 163 |
| 402 | M16112_g_at | Q63094 | 165 |
| 403 | rc_AI232024_f_at | P02563* | 166 |
| 404 | rc_AI180281_at | 008623 | 167 |
| 405 | rc_AI180442_at | P05369 | 168 |
| 406 | rc_AI227677_at | Q62940 | 169 |
| 407 | rc_AI230247_s_at | P25236 | 170 |
| 408 | rc_AI230319_at | P39948 | 171 |
| 409 | rc_Al231572_at | AAP29778 | 174 |
| 410 | rc_Al237371_at | Q9QX82 | 177 |
| 411 | rc_AI171376_at | Q925F0* | 181 |
| 412 | rc_AI172006_at | Q9QX75 | 186 |
| 413 | rc_AI172423_at | AAP12535 | 187 |
| 414 | rc_AI179243_at | Q63503 | 190 |
| 415 | rc_AI178921_s_at | P35559 | 192 |
| 416 | rc_AI175539_at | P02625* | 195 |
| 417 | rc_AI175935_at | Q63356* | 196 |
| 418 | rc_AI176584_at | P24594 | 197 |
| 419 | rc_AI178559_at | P39950 | 198 |
| 420 | rc_AI639233_s_at | Q01129 | 199 |
| 421 | X70871_at | P39950 | 201 |
| 422 | X52311_at | P18395 | 202 |
| 423 | X53504cds_at | P23358 | 203 |
| 424 | X53504cds_g_at | P23358 | 204 |
| 425 | X56133_at | P15999* | 205 |
| 426 | X60351cds_s_at | P23928 | 206 |
| 427 | X64401cds_s_at | P04800 | 207 |
| 428 | X70369_s_at | P13941 | 209 |
| 429 | X04267_at | Q9QZV8* | 210 |
| 430 | X74835cds_at | P25110 | 211 |
| 431 | X76489cds_g_at | P40241 | 212 |
| 432 | X78848cds_f_at | Q9JLX3 | 213 |
| 433 | X80130cds_f_at | P04270* | 214 |
| 434 | Z78279_g_at | Q63079 | 215 |
| 435 | Z83869cds_at | 008679 | 216 |
| 436 | X64827cds_s_at | P16221* | 217 |
| 437 | U20195_s_at | P38652 | 218 |
| 438 | rc_AI639465_f_at | Q91Z63* | 222 |
| 439 | rc_H33725_at | Q8R424 | 224 |
| 440 | X15939_f_at | P02564* | 225 |
| 441 | S74265_s_at | P15146 | 226 |
| 442 | X12554cds_s_at | P10817 | 227 |
| 443 | U25651_at | P47858* | 228 |
| 444 | U30938_at | P15146 | 229 |
| 445 | U40836mRNA_s_at | P16221 | 230 |
| 446 | U50736_s_at | Q8R560 | 231 |
| 447 | U84727_at | P97700 | 232 |
| 448 | U96130_at | 008730 | 233 |
| 449 | S49760_at | 140866 | 235 |
| 450 | rc_AI010583_at | Q8R4I6 | 237 |
| 451 | rc_AA996612_at | Q9Z2J4 | 245 |
| 452 | rc_AA946469_at | AAP29778 | 253 |
| 453 | rc_AA925664_at | 008813 | 257 |
| 454 | rc_AA944560_at | AAN15275 | 259 |
| 455 | rc_AI010562_at | Q63350* | 260 |
| 456 | rc_AA997341_at | Q62920 | 262 |
| 457 | rc_AA946108_at | P70570 | 270 |
| 458 | rc_AI104924_f_at | P02563* | 272 |
| 459 | rc_AI059955_s_at | 008839 | 273 |
| 460 | rc_AI145367_at | P52481 | 279 |
| 461 | rc_AI170777_at | Q9ER34 | 282 |
| 462 | rc_AI170777_g_at | Q9ER34 | 283 |
| 463 | rc_AI170985_at | NP_064489* | 286 |
| 464 | rc_AI171098_at | Q63518* | 287 |
| 465 | rc_AI071299_at | 008876 | 290 |
| 466 | rc_AI111401_s_at | 035217 | 295 |
| 467 | rc_AI102103_g_at | 008561 | 296 |
| 468 | rc_AI103473_at | Q925T0 | 297 |
| 469 | rc_Al103920_f_at | P02563* | 299 |
| 470 | rc_Al104567_g_at | P03996* | 305 |

| | | | |
|---|---|---|---|
| * Muscle-specific ** SPTREMBL | | | |

**Table 4. Human DNA SMPs**

| **SEQ ID NO:** | **AffyID^{™}** | **Accession Number** | **Table 2 SEQ ID NO:** |
|---|---|---|---|
| 471 | rc_Al639444_at | NM_006063.1* | 1 |
| 472 | rc_AA946094_at | NM_005368.1 | 2 |
| 473 | rc_AA891522_f_at | NM 000257.1 * | 3 |
| 474 | rc_AA799471_at | 2330600 | 4 |
| 475 | M24393_at | NM_002479.2* | 7 |
| 476 | M98819mRNA_s_at | NM_000036 | 8 |
| 477 | rc_Al639444_g_at | NM_006063.1* | 12 |
| 478 | rc_AI012182_s_at | NM_000518.4 | 15 |
| 479 | L04684_at | NM_000719 | 16 |
| 480 | rc_AA818804_at | 7022045 | 18 |
| 481 | M12098_s_at | NM_002470.1* | 20 |
| 482 | M27151_at | NM_002469.1* | 22 |
| 483 | AF077338_at | NM_004997.1* | 25 |
| 484 | X74832cds_at | NM_000079.1* | 26 |
| 485 | rc_AA866452_s_at | BC009978* | 28 |
| 486 | AA108284_at | NM_005181.2* | 29 |
| 487 | rc_AI104913_at | NM_003275.1* | 30 |
| 488 | rc_AA818120_at | 1943766 | 31 |
| 489 | AF039832_g_at | NM_000325.3 | 33 |
| 490 | X15939_i_at | NM_000257.1* | 34 |
| 491 | X14812_at | NM_000258.1* | 37 |
| 492 | X15939_r_at | NM_000257.1* | 39 |
| 493 | rc_AA924417_f_at | NM_014332.1* | 42 |
| 494 | X81193_at | NM_003476.1* | 46 |
| 495 | X80130cds_i_at | BC009978 | 48 |
| 496 | rc_AA819140_at | NM_005181.2* | 50 |
| 497 | rc_AI170696_at | NM_016250.1 | 51 |
| 498 | rc_AA892801_at | NM_001961.2 | 61 |
| 499 | rc_AA892287_at | NM_018653 | 66 |
| 500 | rc_AA892313_at | NM_003193 | 67 |
| 501 | rc_AA892468_g_at | NM_002773.2 | 69 |
| 502 | rc_AA859829_g_at | NM_005882 | 70 |
| 503 | rc_AA892468_at | NM_002773.2 | 77 |
| 504 | X96437mRNA_g_at | NM_002727 | 79 |
| 505 | Y09453cds_at | NM_000727.2 | 80 |
| 506 | Z78279_at | BC036531 | 81 |
| 507 | rc_AA875206_at | 222989_s_at | 85 |
| 508 | rc_AA859931_g_at | NM_024069 | 87 |
| 509 | rc_AA859335_at | NM_005507.1* | 93 |
| 510 | K03467_s_at | NM_002470.1* | 96 |
| 511 | H32169_at | BC018256 | 97 |
| 512 | J00692_at | NM_009606* | 99 |
| 513 | J04993_at | NM_003281.2* | 102 |
| 514 | L11694_at | NM_002633.2 | 103 |
| 515 | K02423cds_s_at | NM_079420.1* | 104 |
| 516 | D38056_at | NM_004428.2 | 105 |
| 517 | L00088exp_cds#2_at | NM_079420.1* | 106 |
| 518 | L00382cds_at | X06825* | 107 |
| 519 | L01702_at | NM_002836.2 | 108 |
| 520 | L01793_at | NM_004130.2 | 109 |
| 521 | L01793_g_at | NM_004130.2 | 110 |
| 522 | K02111_at | NM_002470* | 112 |
| 523 | AF052540_s_at | NM_000070.2 | 113 |
| 524 | AB000216_at | NM_145804.1 | 115 |
| 525 | AB009999_g_at | NM_001263.2 | 116 |
| 526 | AF002281_at | NM_014476.1 | 117 |
| 527 | AF008439_at | NM_000617.1 | 118 |
| 528 | AF013144_at | NM_004419.2 | 119 |
| 529 | E12625cds_at | NM_006745.2 | 120 |
| 530 | AF037072_at | NM_005181.2* | 121 |
| 531 | D64046_at | NM_005027.1 | 122 |
| 532 | AF061726_s_at | NM_000070.2* | 123 |
| 533 | AF077338_g_at | NM_004997.1* | 124 |
| 534 | AF093536_at | BC047677 | 127 |
| 535 | D37920_at | NM_003129.2 | 128 |
| 536 | L13606_at | XM_028522* | 129 |
| 537 | AF030089UTR#1_at | NM_004734.1 | 130 |
| 538 | M83298_g_at | NM_002717.2 | 132 |
| 539 | M83676_at | 5410327 | 133 |
| 540 | M84176_at | NM_002478* | 134 |
| 541 | M86621_at | NM_000722.1 | 135 |
| 542 | M89945mRNA_g_at | NM_004462 | 136 |
| 543 | rc_AA799571_at | 13491977 | 137 |
| 544 | L08505_at | NM_001376.2 | 138 |
| 545 | rc_AA800221_at | NM_014332.1* | 139 |
| 546 | M57263_at | NM_000359.1 | 140 |
| 547 | rc_AA817975_at - | NM_005662.3 | 145 |
| 548 | rc_AA818745_at | 29488 | 146 |
| 549 | M21759mRNA_at | XM_048104 | 148 |
| 550 | L24897_s_at | XM_028522* | 149 |
| 551 | L27124_s_at | NM_002525.1 | 150 |
| 552 | L28818cds_at | BC046391 | 151 |
| 553 | M10140_at | NM_001824.2* | 152 |
| 554 | M16112_at | NM_001220.3 | 154 |
| 555 | M63122_at | NM_001065.2 | 155 |
| 556 | M18330_at | NM_006254.2 | 156 |
| 557 | M62752_at | NM_001958.2 | 157 |
| 558 | M23995_g_at | NM_000692 | 158 |
| 559 | M32397_at | NM_001099.2 | 160 |
| 560 | M37941mRNA_s_at | NM_000036.1 | 161 |
| 561 | M37942exn#2-3_s_at | NM_000036 | 162 |
| 562 | M55534mRNA_s_at | NM_001885.1 | 163 |
| 563 | M16112_g_at | NM_001220.3 | 165 |
| 564 | rc_AI232024_f_at | NM_002471.1* | 166 |
| 565 | rc_AI180442_at | NM_002004.1 | 168 |
| 566 | rc_AI227677_at | D42055.1 | 169 |
| 567 | rc_AI230247_s_at | NM_005410.1 | 170 |
| 568 | rc_AI230319_at | NM_001758.1 | 171 |
| 569 | rc_AI237371_at | NM_006016.3 | 177 |
| 570 | rc_AI171376_at | NM_014332.1* | 181 |
| 571 | rc_AI172006_at | NM_032467 | 186 |
| 572 | rc_AI179243_at | NM_021724.1 | 190 |
| 573 | rc_AI178921_s_at | NM_004969.1 | 192 |
| 574 | rc_AI175539_at | NM_002854.1* | 195 |
| 575 | rc_AI175935_at | NM_004998.1* | 196 |
| 576 | rc_AI176584_at | NM_000599.1 | 197 |
| 577 | rc_AI178559_at | NM_004060.2 | 198 |
| 578 | rc_AI639233_s_at | NM_001920.2 | 199 |
| 579 | X70871_at | NM_004060.2 | 201 |
| 580 | X52311_at | NM_002524.2 | 202 |
| 581 | X56133_at | NM_004046.3* | 205 |
| 582 | X60351cds_s_at | NM_001885.1 | 206 |
| 583 | X64401cds_s_at | BC003642 | 207 |
| 584 | X70369_s_at | NM_000090.2 | 209 |
| 585 | X04267_at | NM_002470.1* | 210 |
| 586 | X74835cds_at | NM_000751.1 | 211 |
| 587 | X76489cds_g_at | NM_001769.2 | 212 |
| 588 | X78848cds_f_at | NM_000847.3 | 213 |
| 589 | X80130cds_f_at | BC009978* | 214 |
| 590 | Z78279_g_at | BC036531 | 215 |
| 591 | Z83869cds_at | NM_004954.2 | 216 |
| 592 | X64827cds_s_at | J04823* | 217 |
| 593 | U20195_s_at | NM_002633.2 | 218 |
| 594 | rc_Al639324_at | NM_030793 | 219 |
| 595 | rc_Al639465_f_at | NM_032588.2* | 222 |
| 596 | rc_H33725_at | NM_006463.2 | 224 |
| 597 | X15939_f_at | NM_000257.1* | 225 |
| 598 | S74265_s_at | NM_002374.2 | 226 |
| 599 | X12554cds_s at | NM_005205.2 | 227 |
| 600 | U25651_at | NM_000289.2* | 228 |
| 601 | U30938_at | NM_002374.2 | 229 |
| 602 | U40836mRNA_s_at | 1311703 | 230 |
| 603 | U50736_s_at | NM_014391.1 | 231 |
| 604 | U84727_at | NM_003562.3 | 232 |
| 605 | U96130_at | NM_004130.2 | 233 |
| 606 | S49760_at | BC043292 | 235 |
| 607 | rc_AI010583_at | NM_001104.1 | 237 |
| 608 | rc_AI011709_at | 35526 | 241 |
| 609 | rc_AA996612_at | NM_144573.1 | 245 |
| 610 | rc_AA925122_at | NM_000363 | 255 |
| 611 | rc_AA925664_at | NM_001664 | 257 |
| 612 | rc_AA944560_at | NM_007066.3 | 259 |
| 613 | rc_AA997341_at | NM_006457.1 | 262 |
| 614 | rc_AA946108_at | NM_173306 | 270 |
| 615 | rc_AI168935_at | NM_018286 | 271 |
| 616 | rc_Al04924_f_at | NM_002471.1 * | 272 |
| 617 | rc_Al059955_s_at | NM_004305.2 | 273 |
| 618 | rc_Al145367_at | NM_006366.1 | 279 |
| 619 | rc_Al170777_at | NM_001098.1 | 282 |
| 620 | rc_Al170777_g_at | NM_001098.1 | 283 |
| 621 | rc_AI170894_at | NM_001122 | 288 |
| 622 | rc_AI170985_at | NM_079420.1* | 300 |
| 623 | rc_AI103376_at | NM_018112 | 282 |
| 624 | rc_AI071299_at | NM_005655.1 | 235 |
| 625 | rc_AI111401_s_at | NM_004897.2 | 289 |
| 626 | rc_AI102103_g_at | NM_002651.1 | 278 |
| 627 | rc_AI103473_at | NM_018664.1 | 237 |
| 628 | rc_AI103920_f_at | NM_002471.1* | 281 |
| 629 | rc_AI104354_at | NM_016599 | 93 |
| 630 | rc_AI104567_g_at | BC009978* | 200 |

| | | | |
|---|---|---|---|
| * Muscle-specific | | | |

**Table 5. Human Protein SMPs**

| **SEQ ID NO:** | **AffyID^{™}** | **Accession Number** | **Table 2 SEQ ID NO:** | **Table 4 SEO ID NO:** |
|---|---|---|---|---|
| 631 | rc_AI639444_at | AAH06534* | 1 | 471 |
| 632 | rc_AA946094_at | P02144 | 2 | 472 |
| 633 | rc_AA891522_f_at | P12883* | 3 | 473 |
| 634 | rc_AA799471_at | 015273 | 4 | 474 |
| 635 | M24393_at | P15173* | 7 | 475 |
| 636 | M98819mRNA_s_at | NP_000027 | 8 | 476 |
| 637 | rc_Al639444_g_at | AAH06534* | 12 | 477 |
| 638 | rc_AI012182_s_at | P02023 | 15 | 478 |
| 639 | L04684_at | NP_000710 | 16 | 479 |
| 640 | rc_AA818804_at | Q9NWB1 | 18 | 480 |
| 641 | M12098_s_at | P11055* | 20 | 481 |
| 642 | M27151_at | AAH17834* | 22 | 482 |
| 643 | AF077338_at | AAH44226* | 25 | 483 |
| 644 | X74832cds_at | P02708* | 26 | 484 |
| 645 | rc_AA866452_s_at | AAH009987* | 28 | 485 |
| 646 | AA108284_at | P07451* | 29 | 486 |
| 647 | rc_AI104913_at | P28289* | 30 | 487 |
| 648 | rc_AA818120_at | 000631 | 31 | 488 |
| 649 | AF039832_g_at | Q99697 | 33 | 489 |
| 650 | X15939_i_at | P12883* | 34 | 490 |
| 651 | X14812_at | AAH09790* | 37 | 491 |
| 652 | X15939_r_at | P12883* | 39 | 492 |
| 653 | rc_AA924417_f_at | Q9UHP9* | 42 | 493 |
| 654 | X81193_at | P50461* | 46 | 494 |
| 655 | X80130cds_i_at | AAH009987 | 48 | 495 |
| 656 | rc_AA819140_at | P07451* | 50 | 496 |
| 657 | rc_AI170696_at | CAD62321 | 51 | 497 |
| 658 | rc_AA892801_at | P13639 | 61 | 498 |
| 659 | rc_AA892287_at | NP_061123.2 | 66 | 499 |
| 660 | rc_AA892313_at | NP_003184.1 | 67 | 500 |
| 661 | rc_AA892468_g_at | Q16651 | 69 | 501 |
| 662 | rc_AA859829_g_at | NP_005873.1 | 70 | 502 |
| 663 | rc_AA892468_at | Q16651 | 77 | 503 |
| 664 | X96437mRNA_g_at | AAH22313 | 79 | 504 |
| 665 | Y09453cds_at | Q06432 | 80 | 505 |
| 666 | Z78279_at | P02452 | 81 | 506 |
| 667 | rc_AA875206_at | NP_038466.2 | 85 | 507 |
| 668 | rc_AA859931_g_at | NP_076974.1 | 87 | 508 |
| 669 | rc_AA859335_at | P23528* | 93 | 509 |
| 670 | K03467_s_at | P11055* | 96 | 510 |
| 671 | H32169_at | NP_068733.1 | 97 | 511 |
| 672 | J00692_at | NP_033736* | 99 | 512 |
| 673 | J04993_at | AAH12600* | 102 | 513 |
| 674 | L11694_at | AAH19920 | 103 | 514 |
| 675 | K02423cds_s_at | AAH05318* | 104 | 515 |
| 676 | D38056_at | P20827 | 105 | 516 |
| 677 | L00088expanded_cds#2_at | AAH05318* | 106 | 517 |
| 678 | L00382cds_at | CAA29971* | 107 | 518 |
| 679 | L01702_at | AAH27308 | 108 | 519 |
| 680 | L01793_at | P46976 | 109 | 520 |
| 681 | L01793_g_at | P46976 | 110 | 521 |
| 682 | K02111_at | NP_002481* | 112 | 522 |
| 683 | AF052540_s_at | P20807 | 113 | 523 |
| 684 | AB000216_at | Q8N961 | 115 | 524 |
| 685 | AB009999_g_at | Q92903 | 116 | 525 |
| 686 | AF002281_at | 043590 | 117 | 526 |
| 687 | AF008439_at | BAB93467 | 118 | 527 |
| 688 | AF013144_at | Q16690 | 119 | 528 |
| 689 | E12625cds_at | Q15800 | 120 | 529 |
| 690 | AF037072_at | P07451* | 121 | 530 |
| 691 | D64046_at | 000459 | 122 | 531 |
| 692 | AF061726_s_at | P20807* | 123 | 532 |
| 693 | AF077338_g_at | AAH44226* | 124 | 533 |
| 694 | AF093536_at | AAH47677 | 127 | 534 |
| 695 | D37920_at | Q14534 | 128 | 535 |
| 696 | L13606_at | XP_028522* | 129 | 536 |
| 697 | AF030089UTR#1_at | 015075 | 130 | 537 |
| 698 | M83298_g_at | AAH41071 | 132 | 538 |
| 699 | M83676_at | 088386 | 133 | 539 |
| 700 | M84176_at | NP_002469* | 134 | 540 |
| 701 | M86621_at | P54289 | 135 | 541 |
| 702 | M89945mRNA_g_at | NP_004453 | 136 | 542 |
| 703 | rc_AA799571_at | Q9BXS4 | 137 | 543 |
| 704 | L08505_at | BAA20783 | 138 | 544 |
| 705 | rc_AA800221_at | Q9UHP9* | 139 | 545 |
| 706 | M57263_at | AAH34699 | 140 | 546 |
| 707 | rc_AA817975_at | Q9Y277 | 145 | 547 |
| 708 | rc_AA818745_at | Q01484 | 146 | 548 |
| 709 | M21759mRNA_at | XP_048104 | 148 | 549 |
| 710 | L24897_s_at | XP_028522* | 149 | 550 |
| 711 | L27124_s_at | 043847 | 150 | 551 |
| 712 | L28818cds_at | AAH46391 | 151 | 552 |
| 713 | M10140_at | P06732* | 152 | 553 |
| 714 | M16112_at | AAH19070 | 154 | 554 |
| 715 | M63122_at | P19438 | 155 | 555 |
| 716 | M18330_at | AAH43350 | 156 | 556 |
| 717 | M62752_at | Q05639 | 157 | 557 |
| 718 | M23995_g_at | NP_000683 | 158 | 558 |
| 719 | M32397_at | P15309 | 160 | 559 |
| 720 | M37941mRNA_s_at | P23109 | 161 | 560 |
| 721 | M37942exon#2-3_s_at | NP_000027 | 162 | 561 |
| 722 | M55534mRNA_s_at | P02511 | 163 | 562 |
| 723 | M16112_g_at | AAH19070 | 165 | 563 |
| 724 | rc_Al232024_f_at | 060661* | 166 | 564 |
| 725 | rc_Al180442_at | P14324 | 168 | 565 |
| 726 | rc_AI227677_at | P46934 | 169 | 566 |
| 727 | rc_AI230247_s_at | P49908 | 170 | 567 |
| 728 | rc_AI230319_at | AAH23620 | 171 | 568 |
| 729 | rc_AI237371_at | 095413 | 177 | 569 |
| 730 | rc_AI171376_at | Q9UHP9* | 181 | 570 |
| 731 | rc_AI172006_at | NP_115856 | 186 | 571 |
| 732 | rc_AI179243_at | AAA52334 | 190 | 572 |
| 733 | rc_AI178921_s_at | P14735 | 192 | 573 |
| 734 | rc_AI175539_at | P20472* | 195 | 574 |
| 735 | rc_Al176935_at | Q12965* | 196 | 575 |
| 736 | rc_Al178584_at | P24593 | 197 | 576 |
| 737 | rc_AI178559_at | P51959 | 198 | 577 |
| 738 | rc_AI639233_s_at | P07585 | 199 | 578 |
| 739 | X70871_at | P51959 | 201 | 579 |
| 740 | X52311_at | AAH32446 | 202 | 580 |
| 741 | X56133_at. | P25705* | 205 | 581 |
| 742 | X60351cds_s_at | P02511 | 206 | 582 |
| 743 | X64401cds_s_at | AAH03642 | 207 | 583 |
| 744 | X70369_s_at | AAB59383 | 209 | 584 |
| 745 | X04267_at | P11055* | 210 | 585 |
| 746 | X74835cds_at | Q07001 | 211 | 586 |
| 747 | X76489cds_g_at | P21926 | 212 | 587 |
| 748 | X78848cds_f_at | Q16772 | 213 | 588 |
| 749 | X80130cds_f_at | AAH009987* | 214 | 589 |
| 750 | Z78279_g_at | P02452 | 215 | 590 |
| 751 | Z83869cds_at | Q15449 | 216 | 591 |
| 752 | X64827cds_s_at | P10176* | 217 | 592 |
| 753 | U20195_s_at | AAH19920 | 218 | 593 |
| 754 | rc_AI639324_at | NP_110420.1 | 219 | 594 |
| 755 | rc_AI639465_Cat | Q969Q1* | 222 | 595 |
| 756 | rc_H33725_at | 095630 | 224 | 596 |
| 757 | X15939_f_at | P12883* | 225 | 597 |
| 758 | S74265_s_at | P11137 | 226 | 598 |
| 759 | X12554cds_s_at | AAH29818 | 227 | 599 |
| 760 | U25651_at | AAH12799* | 228 | 600 |
| 761 | U30938_at | P11137 | 229 | 601 |
| 762 | U40836mRNA_s_at | P10176 | 230 | 602 |
| 763 | U50736_s_at | Q15327 | 231 | 603 |
| 764 | U84727_at | Q02978 | 232 | 604 |
| 765 | U96130_at | P46976 | 233 | 605 |
| 766 | S49760_at | S12969 | 235 | 606 |
| 767 | rc_AI010583_at | Q08043 | 237 | 607 |
| 768 | rc_AI011709_at | Q15155 | 241 | 608 |
| 769 | rc_AA996612_at | Q96DL0 | 245 | 609 |
| 770 | rc_AA925122_at | TPHUCC | 255 | 610 |
| 771 | rc_AA925664_at | A32342 | 257 | 611 |
| 772 | rc_AA944560_at | Q9Y2B9 | 259 | 612 |
| 773 | rc_AA997341_at | 060705 | 262 | 613 |
| 774 | rc_AA946108_at | A55347 | 270 | 614 |
| 775 | rc_AI168935_at | NP_060756.1 | 271 | 615 |
| 776 | rc_AI104924_f_at | O60661* | 272 | 616 |
| 777 | rc_AI059955_s_at | CAD28496 | 273 | 617 |
| 778 | rc_AI145367_at | P40123 | 279 | 618 |
| 779 | rc_AI170777_at | Q8TAQ6 | 282 | 619 |
| 780 | rc_AI170777_g_at | Q8TAQ6 | 283 | 620 |
| 781 | rc_AI170894_at | NP_001113.1 | 285 | 621 |
| 782 | rc_AI170985_at | AAH05318* | 286 | 622 |
| 783 | rc_AI103376_at | NP_060582.1 | 289 | 623 |
| 784 | rc_AI071299_at | 075411 | 290 | 624 |
| 785 | rc_AI111401_s_at | 095172 | 295 | 625 |
| 786 | rc_AI102103_g_at | 015096 | 296 | 626 |
| 787 | rc_AI103473_at | Q9NR55 | 297 | 627 |
| 788 | rc_AI103920_f_at | 060661* | 299 | 628 |
| 789 | rc_AI104354_at | NP_057683.1 | 303 | 629 |
| 790 | rc_AI104567_g_at | AAH009987* | 305 | 630 |

| | | | | |
|---|---|---|---|---|
| * Muscle-specific | | | | |

**Table 6. Rat DNA SMNs**

| **SEQ ID NO:** | **AffyID^{™}** | **Accession Number** | **Table 4 SEQ ID NO:** |
|---|---|---|---|
| 791 | rc_AA946094_at | NM_021588 | 2 |
| 792 | rc_AA891522_f_at | NM_017240* | 3 |
| 793 | rc_AA799471_at | AA799471 | 4 |
| 794 | rc_AI172339_at | NM_175844 | 5 |
| 795 | M24393_at | NM_017115* | 7 |
| 796 | X07314cds_at | X07314* | 11 |
| 797 | rc_AA799396_at | AA799396 | 13 |
| 798 | rc_AI012182_s_at | NM_033234 | 15 |
| 799 | rc_AA818804_at | AA818804 | 18 |
| 800 | M12098_s_at | NM_012604* | 20 |
| 801 | M27151_at | NM_013172* | 22 |
| 802 | rc_AI227690_at | AI227690 | 23 |
| 803 | rc_AI175100_at | AI175100 | 24 |
| 804 | AF077338_at | NM_031813* | 25 |
| 805 | X74832cds_at | NM_024485* | 26 |
| 806 | AA108284_at | NM_019292* | 29 |
| 807 | rc_AI104913_at | NM_013044* | 30 |
| 808 | rc_AA818120_at | AA818120 | 31 |
| 809 | X59864mRNA_at | X59864 | 32 |
| 810 | AF039832_g_at | NM_019334 | 33 |
| 811 | X15939_i_at | NM_017240* | 34 |
| 812 | rc_AA851497_f_at | AA851497 | 35 |
| 813 | X14812_at | NM_012606* | 37 |
| 814 | X15939_r_at | NM_017240* | 39 |
| 815 | rc_AI170696_at | NM_133583 | 51 |
| 816 | rc_AA892801_at | NM_017245 | 61 |
| 817 | rc_AA875288_at | AA875288 | 62 |
| 818 | rc_AA891938_at | AA891938 | 65 |
| 819 | rc_AA892287_at | AA892287 | 66 |
| 820 | rc_AA892313_at | AA892313 | 67 |
| 821 | rc_AA892468_g_at | NM_138836 | 69 |
| 822 | rc_AA859829_g_at | AA859829 | 70 |
| 823 | rc_AA892860_g_at | AA892860 | 71 |
| 824 | rc_AA893307_at | AA893307 | 75 |
| 825 | rc_AA894101_g_at | AA894101 | 76 |
| 826 | Z78279_at | Z78279 | 81 |
| 827 | rc_AI172054_at | AI172054 | 82 |
| 828 | rc_AA875206_at | NM_053747 | 85 |
| 829 | rc_AI172189_at | AI172189 | 86 |
| 830 | rc_AA859931_g_at | AA859931 | 87 |
| 831 | rc_AA858921_at | AA858921 | 92 |
| 832 | rc_AA859335_at | NM_017147* | 93 |
| 833 | H32169_at | H32169 | 97 |
| 834 | J01435cds#8_s_at | J01435 | 100 |
| 835 | J01436cds_s_at | J01436 | 101 |
| 836 | J04993_at | NM_017184* | 102 |
| 837 | L11694_at | NM_017033 | 103 |
| 838 | D38056_at | NM_053599 | 105 |
| 839 | L01702_at | NM_012763 | 108 |
| 840 | AB009999_g_at | NM_031242 | 116 |
| 841 | AF008439_at | NM_0131 73 | 118 |
| 842 | AF037072_at | NM_019292* | 121 |
| 843 | D64046_at | NM_0221 85 | 122 |
| 844 | AF077338_g_at | NM_031813* | 124 |
| 845 | AF080507_at | AF080507 | 125 |
| 846 | D37920_at | NM_0171 36 | 128 |
| 847 | M83298_g_at | NM_053999 | 132 |
| 848 | M83676_at | M83676 | 133 |
| 849 | rc_AA799571_at | AA799571 | 137 |
| 850 | M57263_at | NM_031659 | 140 |
| 851 | rc_AA817975_at | NM_031355 | 145 |
| 852 | rc_AA818745_at | AA818745 | 146 |
| 853 | L27124_s_at | NM_012993 | 150 |
| 854 | M18330_at | NM_133307 | 156 |
| 855 | M32397_at | NM_020072 | 160 |
| 856 | rc_AI227677_at | AI227677 | 169 |
| 857 | rc_AI230247_s_at | NM_0191 92 | 170 |
| 858 | rc_AI230596_at | AI230596 | 172 |
| 859 | rc_AI638960_at | AI638960 | 179 |
| 860 | rc_AI171376_at | NM_0533 95* | 181 |
| 861 | rc_AI172423_at | NM_1813 68 | 187 |
| 862 | rc_AI178921_s_at | NM_0131 59 | 192 |
| 863 | rc_AI175935_at | NM_1731 01* | 196 |
| 864 | rc_AI176584_at | NM_012817 | 197 |
| 865 | X52311_at | NM_0540 06 | 202 |
| 866 | X53504cds_g_at | X53504 | 204 |
| 867 | X64401cds_s_at | NM_173144 | 207 |
| 868 | X76489cds_g_at | X76489 | 212 |
| 869 | Z78279_g_at | Z78279 | 215 |
| 870 | Z83869cds_at | NM_021699 | 216 |
| 871 | X64827cds_s_at | NM_012786* | 217 |
| 872 | rc_Al639324_at | Al639324 | 219 |
| 873 | rc_Al639410_i_at | Al639410 | 220 |
| 874 | rc_Al639465_f_at | NM_080903* | 222 |
| 8.75 | rc_H33725_at | NM_138531 | 224 |
| 876 | X12554cds_s_at | NM_012812 | 227 |
| 877 | U30938_at | NM_013066 | 229 |
| 878 | U40836mRNA_s_at | NM_012786 | 230 |
| 879 | S49760_at | NM_080787 | 235 |
| 880 | rc_AI011709_at | AI011709 | 241 |
| 881 | rc_AI043640_at | AI043640 | 248 |
| 882 | rc_AI044292_s_at | AI044292 | 249 |
| 883 | rc_AA925122_at | AA925122 | 255 |
| 884 | rc_AA925664_at | AA925664 | 257 |
| 885 | rc_AA946108_at | NM_173306 | 270 |
| 886 | rc_AI168935_at | AI168935 | 271 |
| 887 | rc_AA924428_at | AA924428 | 278 |
| 888 | rc_AI170894_at | AI170894 | 285 |
| 889 | rc_AI103376_at | AI103376 | 289 |
| 890 | rc_Al071299_at | NM_031135 | 290 |
| 891 | rc_Al072166_at | A1072166 | 293 |
| 892 | rc_AI111401_s_at | AI111401 | 295 |
| 893 | rc_AI103507_at | AI103507 | 298 |
| 894 | rc_AI104349_at | AI104349 | 302 |
| 895 | rc_AI104354_at | AI104354 | 303 |
| 896 | rc_AA892861_at | AA892861 | 306 |
| 897 | rc_AI179358_at | AI179358 | 307 |

| | | | |
|---|---|---|---|
| * Musde-spedfic | | | |

**Table 7. Rat Protein SMPs**

| **SEQ ID NO:** | **AffyID^{™}** | **Table SEQ ID NO:** | **6** | **Table 3 SEQ ID NO:** |
|---|---|---|---|---|
| 898 | rc_AA946094_at | Q9QZ76 | 2 | 791 |
| 899 | rc_AA891522_f_at | P02564* | 3 | 792 |
| 900 | rc_AI172339_at | Q8K4K7 | 5 | 794 |
| 901 | M24393_at | P20428* | 7 | 795 |
| 902 | X07314cds_at | P08733* | 11 | 796 |
| 903 | rc_AI012182_s_st | P02091 | 15 | 798 |
| 904 | M12098_s_at | Q9QZV8* | 20 | 800 |
| 905 | M27151_at | P19335* | 22 | 801 |
| 906 | AF077338_at | 088599* | 25 | 804 |
| 907 | X74832ods_at | P25108* | 26 | 805 |
| 908 | AA108284_at | P14141* | 29 | 806 |
| 909 | rc_AI104913_at | P70567* | 30 | 807 |
| 910 | X59864mRNA_at | Q03668** | 32 | 809 |
| 911 | AF039832_g_at | Q9R0W1 | 33 | 810 |
| 912 | X15939_i_at | P02564* | 34 | 811 |
| 913 | X14812_at | P16409* | 37 | 813 |
| 914 | X15939_r_at | P02564* | 39 | 814 |
| 915 | rc_AI170696_at | Q8VBU2 | 51 | 815 |
| 916 | rc_AA892801_at | P05197 | 61 | 816 |
| 917 | rc_AA892468_g_at | Q9ES87 | 69 | 821 |
| 918 | Z78279_at | Q63079 | 81 | 826 |
| 919 | rc_AA875206_at | Q9JJP9 | 85 | 828 |
| 920 | rc_AA859335_at | P45592* | 93 | 832 |
| 921 | J01436ods_s_at | AAA99907 | 101 | 835 |
| 922 | J04993_at | P13413* | 102 | 836 |
| 923 | L11694_at | P38652 | 103 | 837 |
| 924 | D38056_at | P97553 | 105 | 838 |
| 925 | L01702_at | Q03348 | 108 | 839 |
| 926 | AB009999_g_at | O35052 | 116 | 840 |
| 927 | AF008439_at | 054902 | 118 | 841 |
| 928 | AF037072_at | P14141* | 121 | 842 |
| 929 | D64046_at | Q63788 | 122 | 843 |
| 930 | AF077338_g_at | 088599* | 124 | 844 |
| 931 | D37920_at | P52020 | 128 | 846 |
| 932 | M83298_g_at | P36876 | 132 | 847 |
| 933 | M83676_at | P35284 | 133 | 848 |
| 934 | M57263_at | P23606 | 140 | 850 |
| 935 | rc_AA817975_at | Q9R1Z0 | 145 | 851 |
| 936 | L27124_s_at | 25499 | 150 | 853 |
| 937 | M18330_at | 170538 | 156 | 854 |
| 938 | M32397_at | P20646 | 160 | 855 |
| 939 | rc_Al227677_at | Q62940 | 169 | 856 |
| 940 | rc_Al230247_s_at | P25236 | 170 | 857 |
| 941 | rc_Al171376_at | Q925F0* | 181 | 860 |
| 942 | rc_Al172423_at | AAP12535 | 187 | 861 |
| 943 | rc_Al178921_s_at | P35559 | 192 | 862 |
| 944 | rc_A1175935_at | Q63356* | 196 | 863 |
| 945 | rc_Al176584_at | P24594 | 197 | 864 |
| 946 | X52311_at | P18395 | 202 | 865 |
| 947 | X53504cds_g_at | P23358 | 204 | 866 |
| 948 | X64401cds_s_at | P04800 | 207 | 867 |
| 949 | X76489cds_g_at | P40241 | 212 | 868 |
| 950 | Z78279_g_at | Q63079 | 215 | 869 |
| 951 | Z83869cds_at | 008679 | 216 | 870 |
| 952 | X64827cds_s_at | P16221* | 217 | 871 |
| 953 | rc_Al639465_f_at | Q91Z63* | 222 | 874 |
| 954 | rc_H33725_at | Q8R424 | 224 | 875 |
| 955 | X12554cds_s_at | P10817 | 227 | 876 |
| 956 | U30938_at | P15146 | 229 | 877 |
| 957 | U40836mRNA_s_at | P16221 | 230 | 878 |
| 958 | S49760_at | 140866 | 235 | 879 |
| 959 | rc_AA925664_at | 008813 | 257 | 884 |
| 960 | rc_AA946108_at | P70570 | 270 | 885 |
| 961 | rc_Al071299_at | 008876 | 290 | 890 |
| 962 | rc_Al111401_s_at | 035217 | 295 | 892 |

| | | | | |
|---|---|---|---|---|
| * Muscle-specific ** SPTREMBL | | | | |

**Table 8. Human DNA SMNs**

| **SEQ ID NO: ID** | **AffyID^{™}** | **Accession Number** | **Table 6 SEQ ID NO:** | **Table 4 SEQ ID NO:** |
|---|---|---|---|---|
| 963 | rc_AA946094_at | NM_005368.1 | 2 | 791 |
| 964 | rc_AA891522_f_at | NM_000257.1* | 3 | 792 |
| 965 | rc_AA799471_at | 2330600 | 4 | 793 |
| 966 | M24393_at | NM_002479.2* | 7 | 795 |
| 967 | rc_AI012182_s_at | NM_000518.4 | 15 | 798 |
| 968 | rc_AA818804_at | 7022045 | 18 | 799 |
| 969 | M12098_s_at | NM_002470.1* | 20 | 800 |
| 970 | M27151_at | NM_002469.1* | 22 | 801 |
| 971 | AF077338_at | NM_004997.1* | 25 | 804 |
| 972 | X74832cds_at | NM_000079.1* | 26 | 805 |
| 973 | AA108284_at | NM_005181.2* | 29 | 806 |
| 974 | rc_Al104913_at | NM_003275.1* | 30 | 807 |
| 975 | rc_AA818120_at | 1943766 | 31 | 808 |
| 976 | AF039832_g_at | NM_000325.3 | 33 | 810 |
| 977 | X15939_i_at | NM_000257.1* | 34 | 811 |
| 978 | X14812_at | NM_000258.1* | 37 | 813 |
| 979 | X15939_r_at | NM_000257.1* | 39 | 814 |
| 980 | rc_AI170696_at | NM_016250.1 | 51 | 815 |
| 981 | rc_AA892801_at | NM_001961.2 | 61 | 816 |
| 982 | rc_AA892287_at | NM_018653 | 66 | 819 |
| 983 | rc_AA892313_at | NM_003193 | 67 | 820 |
| 984 | rc_AA892468_g_at | NM_002773.2 | 69 | 821 |
| 985 | rc_AA859829_g_at | NM_005882 | 70 | 822 |
| 986 | Z78279_at | BC036531 | 81 | 826 |
| 987 | rc_AA875206_at | 222989_s_at | 85 | 828 |
| 988 | rc_AA859931_g_at | NM_024069 | 87 | 830 |
| 989 | rc_AA859335_at | NM_005507.1* | 93 | 832 |
| 990 | H32169_at | BC018256 | 97 | 833 |
| 991 | J04993_at | NM_003281.2* | 102 | 836 |
| 992 | L11694_at | NM_002633.2 | 103 | 837 |
| 993 | 038056_at | NM_004428.2 | 105 | 838 |
| 994 | L01702_at | NM_002836.2 | 108 | 839 |
| 995 | AB009999_g_at | NM_001263.2 | 116 | 840 |
| 996 | AF008439_at | NM_000617.1 | 118 | 841 |
| 997 | AF037072_at | NM_005181.2* | 121 | 842 |
| 998 | 064046_at | NM_005027.1 | 122 | 843 |
| 999 | AF077338_g_at | NM_004997.1* | 124 | 844 |
| 1000 | D37920_at | NM_003129.2 | 128 | 846 |
| 1001 | M83298_g_at | NM_002717.2 | 132 | 847 |
| 1002 | M83676_at | 5410327 | 133 | 848 |
| 1003 | rc_AA799571_at | 13491977 | 137 | 849 |
| 1004 | M57263_at | NM_000359.1 | 140 | 850 |
| 1005 | rc_AA817975_at | NM_005662.3 | 145 | 851 |
| 1006 | rc_AA818745_at | 29488 | 146 | 852 |
| 1007 | L27124_s_at | NM_002525.1 | 150 | 853 |
| 1008 | M18330_at | NM_006254.2 | 156 | 854 |
| 1009 | M32397_at | NM_001099.2 | 160 | 855 |
| 1010 | rc_AI227877_at | D42055.1 | 169 | 856 |
| 1011 | rc_AI230247_s_at | NM_005410.1 | 170 | 857 |
| 1012 | rc_AI171376_at | NM_014332.1* | 181 | 860 |
| 1013 | rc_AI178921_s_at | NM_OO4969.1 | 192 | 862 |
| 1014 | rc_AI175935_at | NM_004998.1* | 196 | 863 |
| 1015 | rc_AI176584_at | NM_000599.1 | 197 | 864 |
| 1016 | X52311_at | NM_002524.2 | 202 | 865 |
| 1017 | X64401cds_s_at | BC003642 | 207 | 867 |
| 1018 | X76489cds_g_At | NM_001769.2 | 212 | 868 |
| 1019 | Z78279_g_at | BC036531 | 215 | 869 |
| 1020 | Z83869cds_at | NM_004954.2 | 216 | 870 |
| 1021 | X64827cds_s_at | J04823* | 217 | 871 |
| 1022 | rc_AI639324_at | NM_030793 | 219 | 872 |
| 1023 | rc_AI639465_f_at | NM_032588.2* | 222 | 874 |
| 1024 | rc_H33725_at | NM_006463.2 | 224 | 875 |
| 1025 | X12554cds_s_at | NM_005205.2 | 227 | 876 |
| 1026 | U30938_at | NM_002374.2 | 229 | 877 |
| 1027 | U40836mRNA_s_at | 1311703 | 230 | 878 |
| 1028 | S49760_at | BC043292 | 235 | 879 |
| 1029 | rc_AI011709_at | 35526 | 241 | 880 |
| 1030 | rc_AA925122_at | NM_000363 | 255 | 883 |
| 1031 | rc_AA925664_at | NM_001664 | 257 | 884 |
| 1032 | rc_AA946108_at | NM_173306 | 270 | 885 |
| 1033 | rc_AI168935_at | NM_018286 | 271 | 886 |
| 1034 | rc_AI170894_at | NM_001122 | 285 | 888 |
| 1035 | rc_AI103376_at | NM_018112 | 289 | 889 |
| 1036 | rc_AI071299_at | NM_005655.1 | 290 | 890 |
| 1037 | rc_AI111401_s_at | NM_004897.2 | 295 | 892 |
| 1038 | rc_AI104354_at | NM_016599 | 303 | 895 |

| | | | | |
|---|---|---|---|---|
| * Muscle-speciflc | | | | |

**Table 9. Human Protein SMNs**

| **SEQ ID NO:** | **AffyID^{™}** | **Accession Number** | **Table 8 SEQ ID NO:** | **Table 5 SEQ ID NO:** |
|---|---|---|---|---|
| 1039 | rc_AA946094_at | P02144 | 2 | 963 |
| 1040 | rc_AA891522_f_at | P12883* | 3 | 964 |
| 1041 | rc_AA799471_at | 015273 | 4 | 965 |
| 1042 | M24393_at | P15173* | 7 | 966 |
| 1043 | rc_AI012182_s_at | P02023 | 15 | 967 |
| 1044 | rc_AA818804_at | Q9NWB1 | 18 | 968 |
| 1045 | M12098_s_at | P11055* | 20 | 969 |
| 1046 | M27151_at | AAH17834* | 22 | 970 |
| 1047 | AF077338_at | AAH44226* | 25 | 971 |
| 1048 | X74832cds_at | P02708* | 26 | 972 |
| 1049 | AA108284_at | P07451* | 29 | 973 |
| 1050 | rc_AI104913_at | P28289* | 30 | 974 |
| 1051 | rc_AA818120_at | 000631 | 31 | 975 |
| 1052 | AF039832_g_at | Q99697 | 33 | 976 |
| 1053 | X15939_i_at | P12883* | 34 | 977 |
| 1054 | X14812_at | AAH09790* | 37 | 978 |
| 1055 | X15939_r_at | P12883* | 39 | 979 |
| 1056 | rc_AI170696_at | CAD62321 | 51 | 980 |
| 1057 | rc_AA892801_at | P13639 | 61 | 981 |
| 1058 | rc_AA892287_at | NP_061123.2 | 66 | 982 |
| 1059 | rc_AA892313_at | NP_003184.1 | 67 | 983 |
| 1060 | rc_AA892468_g_at | Q16651 | 69 | 984 |
| 1061 | rc_AA859829_g_at | NP_005873.1 | 70 | 985 |
| 1062 | Z78279_at | P02452 | 81 | 986 |
| 1063 | rc_AA875206_at | NP_038466.2 | 85 | 987 |
| 1064 | rc_AA859931_g_at | NP_076974.1 | 87 | 988 |
| 1065 | rc_AA859335_at | P23528* | 93 | 989 |
| 1066 | H32169_at | NP_068733.1 | 97 | 990 |
| 1067 | J04993_at | AAH12600* | 102 | 991 |
| 1068 | L11694_at | AAH19920 | 103 | 992 |
| 1069 | D38056_at | P20827 | 105 | 993 |
| 1070 | L01702_at | AAH27308 | 108 | 994 |
| 1071 | AB009999_g_at | Q92903 | 116 | 995 |
| 1072 | AF008439_at | BAB93467 | 118 | 996 |
| 1073 | AF037072_at | P07451* | 121 | 997 |
| 1074 | D64046_at | 000459 | 122 | 998 |
| 1075 | AF077338_g_at | AAH44226* | 124 | 999 |
| 1076 | D37920_at | Q14534 | 128 | 1000 |
| 1077 | M83298_g_at | AAH41071 | 132 | 1001 |
| 1078 | M83676_at | 088386 | 133 | 1002 |
| 1079 | rc_AA799571_at | Q9BXS4 | 137 | 1003 |
| 1080 | M57283_at | AAH34699 | 140 | 1004 |
| 1081 | rc_AA817975_at | Q9Y277 | 145 | 1005 |
| 1082 | rc_AA818745_at | Q01484 | 146 | 1006 |
| 1083 | L27124_s_at | 043847 | 150 | 1007 |
| 1084 | M18330_at | AAH43350 | 156 | 1008 |
| 1085 | M32397_at | P15309 | 160 | 1009 |
| 1086 | rc_Al227677_at | P46934 | 169 | 1010 |
| 1087 | rc_Al230247_s_at | P49908 | 170 | 1011 |
| 1088 | rc_AI171376_at | Q9UHP9* | 181 | 1012 |
| 1089 | rc_AI178921_s_at | P14735 | 192 | 1013 |
| 1090 | rc_AI175935_at | Q12965* | 196 | 1014 |
| 1091 | rc_AI176584_at | P24593 | 197 | 1015 |
| 1092 | X52311_at | AAH32446 | 202 | 1016 |
| 1093 | X64401cds_s_at | AAH03642 | 207 | 1017 |
| 1094 | X76489cds_g_at | P21926 | 212 | 1018 |
| 1095 | Z7e279_g_at | P02452 | 215 | 1019 |
| 1096 | Z83869cds_st | Q15449 | 216 | 1020 |
| 1097 | X64827cds_s_st | P10176* | 217 | 1021 |
| 1098 | rc_AI639324_st | NP_110420.1 | 219 | 1022 |
| 1099 | rc_Al639465_f_at | Q969Q1* | 222 | 1023 |
| 1100 | rc_H33725_at | 095630 | 224 | 1024 |
| 1101 | X12554cds_s_at | AAH29818 | 227 | 1025 |
| 1102 | U30938_at | P11137 | 229 | 1026 |
| 1103 | U40836mRNA_s_at | P10176 | 230 | 1027 |
| 1104 | S49760_at | S12969 | 235 | 1028 |
| 1105 | rc_Al011709_at | Q15155 | 241 | 1029 |
| 1106 | rc_AA925122_at | TPHUCC | 255 | 1030 |
| 1107 | rc_AA925664_at | A32342 | 257 | 1031 |
| 1108 | rc_AA946108_at | A55347 | 270 | 1032 |
| 1109 | rc_AI168935_at | NP_060756.1 | 271 | 1033 |
| 1110 | rc_AI170894_at | NP_001113.1 | 285 | 1034 |
| 1111 | rc_AI03376_at | NP_060582.1 | 289 | 1035 |
| 1112 | rc_AI071299_at | 075411 | 290 | 1036 |
| 1113 | rc_AI111401_s_at | 095172 | 295 | 1037 |
| 1114 | rc_AI104354_at | NP_057683.1 | 303 | 1038 |

| | | | | |
|---|---|---|---|---|
| * Muscle-specific | | | | |

**Table 10. Rat DNA BMNs**

| **SEQ ID NO:** | **AffyID^{™}** | **Accession Number** | **Table 2 SEQ ID NO:** |
|---|---|---|---|
| 1115 | rc_Al233261_i_at | NM_017305 | N/A |
| 1116 | rc_Al012354_at | NM_022647 | N/A |
| 1117 | rc_AA955974_at | AA955974 | N/A |
| 1118 | rc_AI072712_at | Al072712 | N/A |
| 1119 | rc_Al029088_at | Al029088 | N/a |
| 1120 | X15939_i_at | NM_017240* | 34 |
| 1121 | rc_AA850730_at | AA850730 | N/A |
| 1122 | rc_AA998245_at | AA998245 | N/A |
| 1123 | rc_AI233173_at | NM_138548 | N/A |
| 1124 | rc_AI232350_f_at | AI232350 | N/A |
| 1125 | rc_AA998683_at | NM_031970 | N/A |
| 1126 | rc_AA858921_at | AA858921 | 92 |
| 1127 | rc_AA850705_at | NM_153309 | N/A |
| 1128 | rc_AA998097_at | AA998097 | N/A |
| 1129 | rc_AA997841_at | NM_133298 | N/A |
| 1130 | rc_AA848449_at | AA848449 | N/A |
| 1131 | rc_AA819268_at | AA819268 | N/A |
| 1132 | rc_AA800268_at | AA800268 | N/A |
| 1133 | M14050_s_at | NM_013083 | N/A |
| 1134 | L06804_at | L06804 | N/A |
| 1135 | D12769_at | NM_057211 | N/A |
| 1136 | AF036335_g_at | AF036335 | N/A |
| 1137 | AA801076_at | AA801076 | N/A |
| 1138 | rc_AA848829_at | AA848829 | N/A |
| 1139 | rc_Al030259_at | Al030259 | N/A |
| 1140 | S69316_s_at | S69316 | N/A |
| 1141 | rc_AI639155_at | AI639155 | N/A |
| 1142 | rc_AI639012_at | AI639012 | N/A |
| 1143 | rc_AI237654_at | AI237654 | N/A |
| 1144 | rc_AI228696_at | AI228696 | N/A |
| 1145 | rc_AI177055_at | AI177055 | N/A |
| 1146 | rc_AI176969_at | AI176969 | N/A |
| 1147 | rc_AI102438_at | AI102438 | N/A |
| 1148 | rc_AI072603_at | AI072603 | N/A |
| 1149 | rc_AI059519_at | AI059519 | N/A |
| 1150 | rc_AA899491_at | AA899491 | N/A |
| 1151 | rc_AI044635_at | AI044635 | N/A |
| 1152 | rc_AA874873_g_at | AA874873 | N/A |
| 1153 | rc_AI014091_at | NM_053698 | N/A |
| 1154 | rc_AI013854_at | AI013854 | N/A |
| 1155 | rc_AI012937_at | AI012937 | N/A |
| 1156 | rc_AI008911_at | Al008911 | N/A |
| 1157 | rc_AI007875_at | NM_053435 | N/A |
| 1158 | rc_AI007672_at | Al007672 | N/A |
| 1159 | rc_AA997726_at | AA997726 | N/A |
| 1160 | rc_AA963457_at | AA963457 | N/A |
| 1161 | rc_AA963171_at | AA963171 | N/A |
| 1162 | rc_AA924573_s_at | AA924573 | N/A |
| 1163 | rc_AI058912_at | A1058912 | N/A |

| | | | |
|---|---|---|---|
| * Muscle-specfic | | | |

**Table 11. Rat Protein BMNs**

| **SEQ ID No.** | **AffyID^{™}** | **Accessio n Number** | **Table 10 SEQ ID NO:** | **Table 2 SEQ ID NO:** |
|---|---|---|---|---|
| 1164 | rc_AI233261_i_at | P48508 | 1115 | N/A |
| 1165 | rc_AI012354_at | 64647 | 1116 | N/A |
| 1166 | X15939_i_at | P02564* | 1120 | 34 |
| 1167 | rc_AI233173_at | Q05982 | 1123 | N/A |
| 1168 | rc_AA998683_at | P42930 | 1125 | N/A |
| 1169 | rc_AA850705_at | Q8CFC1 | 1127 | N/A |
| 1170 | rc_AA997841_at | Q9QZF6 | 1129 | N/A |
| 1171 | M14050_s_at | P06761 | 1133 | N/A |
| 1172 | L06804_at | P36198 | 1134 | N/A |
| 1173 | D12769_at | Q01713 | 1135 | N/A |
| 1174 | AF036335_g_at | 054725 | 1136 | N/A |
| 1175 | S69316_s_at | S69316 | 1140 | N/A |
| 1176 | rc_AI237654_at | 117514 | 1143 | N/A |
| 1177 | rc_AI014091_at | Q99MA1 | 1153 | N/A |
| 1178 | rc_At007875_at | Q9ESZ0 | 1157 | N/A |

| | | | | |
|---|---|---|---|---|
| * Muscle-specific | | | | |

**Table 12 Human DNA BMNs.**

| **SEQ ID NO:** | **AffyID^{™}** | **Accession Number** | **Table 10 SEQ ID NO:** | **Table 4 SEQ ID NO:** |
|---|---|---|---|---|
| 1179 | rc_AI233261_i_at | NM_002061.1 | 1115 | N/A |
| 1180 | rc_AI012354_at | NM_022647 | 1116 | N/A |
| 1181 | rc_AA955974_at | NM_001851 | 1117 | N/A |
| 1182 | X15939_i_at | NM_000257.1* | 1120 | 490 |
| 1183 | rc_AA850730_at | 505095 | 1121 | N/A |
| 1184 | rc_AI233173_at | NM_000269.1 | 1123 | N/A |
| 1185 | rc_AA998683_at | NM_031970 | 1125 | N/A |
| 1186 | rc_AA850705_at | 213189_at | 1127 | N/A |
| 1187 | rc_AA998097_at | 1000283 | 1128 | N/A |
| 1188 | rc_AA997841_at | NM_002510.1 | 1129 | N/A |
| 1189 | rc_AA800268_at | NM_014182 | 1132 | N/A |
| 1190 | M14050_s_at | NM_005347.2 | 1133 | N/A |
| 1191 | L06804_at | 600494 | 1134 | N/A |
| 1192 | D12769_at | NM_001206.1 | 1135 | N/A |
| 1193 | AF036335_g_at | NM_005066.1 | 1136 | N/A |
| 1194 | rc_Al030259_at | NM_031219 | 1139 | N/A |
| 1195 | rc_Al639012_at | NM_024042 | 1142 | N/A |
| 1196 | rc_AI237654_at | NM_006472.1 | 1143 | N/A |
| 1197 | rc_AI228696_at | NM_012106 | 1144 | N/A |
| 1198 | rc_AI177055_at | NM_053050 | 1145 | N/A |
| 1199 | rc_AI059519_at | NM_024324 | 1149 | N/A |
| 1200 | rc_AA899491_at | XM_291885 | 1150 | N/A |
| 1201 | rc_AI044635_at | NM_014934 | 1151 | N/A |
| 1202 | rc_AI014091_at | NM_006079.2 | 1153 | N/A |
| 1203 | rc_AI012937_at | NM_013442 | 1155 | N/A |
| 1204 | rc_AI008911_at | NM_012470 | 1156 | N/A |
| 1205 | rc_AI007875_at | 203655_at | 1157 | N/A |
| 1206 | rc_AA997726_at | 1136429 | 1159 | N/A |
| 1207 | rc_AA963171_at | NM_031210 | 1161 | N/A |

| | | | | |
|---|---|---|---|---|
| * Muscle-specific | | | | |

**Table 13. Human Protein BMNs**

| **SEQ ID NO:** | **AffyID^{™}** | **Accession Number** | **Table 12 SEQ ID NO:** | **Table 5 SEQ ID** |
|---|---|---|---|---|
| 1208 | rc_AI233261_i_at | AAH41809 | 1115 | 1179 |
| 1209 | rc_AI012354_at | E40335 | 1116 | 1180 |
| 1210 | rc_AA955974_at | NP_001842.2 | 1117 | 1181 |
| 1211 | X15939_i_at | P12883* | 1120 | 1182 |
| 1212 | rc_AA850730_at | Q15040 | 1121 | 1183 |
| 1213 | rc_AI233173_at | P15531 | 1123 | 1184 |
| 1214 | rc_AA998683_at | HHHU27 | 1125 | 1185 |
| 1215 | rc_AA850705_at | NP_116167.1 | 1127 | 1186 |
| 1216 | rc_AA998097_at | P49903 | 1128 | 1187 |
| 1217 | rc_AA997841_at | Q14956 | 1129 | 1188 |
| 1218 | rc_AA800268_at | NP_054901.1 | 1132 | 1189 |
| 1219 | M14050_s_at | AAH20235 | 1133 | 1190 |
| 1220 | L06804_at | P50458 | 1134 | 1191 |
| 1221 | D12769_at | Q13886 | 1135 | 1192 |
| 1222 | AF036335_g_at | P23246 | 1136 | 1193 |
| 1223 | rc_AI030259_at | NP_112496.1 | 1139 | 1194 |
| 1224 | rc_AI639012_at | NP_076947.1 | 1142 | 1195 |
| 1225 | rc_AI237654_at | Q16226 | 1143 | 1196 |
| 1226 | rc_AI228696_at | NP_036238.1 | 1144 | 1197 |
| 1227 | rc_AI177055_at | NP_444278.1 | 1145 | 1198 |
| 1228 | re_AI059519_at | NP_077300.1 | 1149 | 1199 |
| 1229 | rc_AA899491_at | A41706 | 1150 | 1200 |
| 1230 | rc_AI044635_at | NP_055749.1 | 1151 | 1201 |
| 1231 | rc_AI014091_at | Q99967 | 1153 | 1202 |
| 1232 | rc_AI012937_at | NP_038470.1 | 1155 | 1203 |
| 1233 | rc_AI008911_at | NP_036602.1 | 1156 | 1204 |
| 1234 | rc_AI007875_at | A36353 | 1157 | 1205 |
| 1235 | rc_AA997726_at | Q14690 | 1159 | 1206 |
| 1236 | rc_AA963171_at | NP_112487.1 | 1161 | 1207 |

| | | | | |
|---|---|---|---|---|
| * Muscle-specific | | | | |

**Table 16. Additional Rat DNA BMNs**

| **SEQ ID NO:** | **AffyID^{™}** | **Accession Number** |
|---|---|---|
| 1252 | M74494_g_at | M74494 |
| 1253 | X74565cds_at | NM_022516 |
| 1254 | X74565cds_g_at | NM_022516 |
| 1255 | rc_AI175833_at | NM_001007005 |
| 1256 | rc_AA900425_s_at | NM_022516 |
| 1257 | rc_AA998722_s_at | BC061541 |
| 1258 | rc_AI104953_s_at | NM_139106 |
| 1259 | rc_AI028975_s_at | NM_017277 |
| 1260 | X13016_at | NM_139103 |
| 1261 | rc_AA926149_g_at | NM_012520 |
| 1262 | rc_AI169756_s_at | Al169756 |
| 1263 | H32867_at | XM_216192 |
| 1264 | rc_AI227743_at | XM_238333 |

**Table 17. Additional Rat Protein BMNs**

| **SEQ ID NO:** | **Accession Number** | **Table 16 SEQ ID NO:** |
|---|---|---|
| 1265 | P06685 | 1252 |
| 1266 | Q00438 | 1253 |
| 1267 | Q00438 | 1254 |
| 1268 | Q5XI73 | 1255 |
| 1269 | Q00438 | 1256 |
| 1270 | P11980 | 1257 |
| 1271 | P35434 | 1258 |
| 1272 | P52303 | 1259 |
| 1273 | P10252 | 1260 |
| 1274 | P04762 | 1261 |
| 1275 | Q5XIG7 | 1264 |

**Table 18. Additional Human DNA BMNs**

| **SEQ ID NO:** | **Accession Number** | **Table 16 SEQ ID NO:** |
|---|---|---|
| 1276 | NM_000701 | 1252 |
| 1277 | NM_031990 | 1253 |
| 1278 | NM_031990 | 1254 |
| 1279 | NM_004309 | 1255 |
| 1280 | NM_031990 | 1256 |
| 1281 | NM_002654 | 1257 |
| 1282 | BC004426 | 1258 |
| 1283 | NM_001127 | 1259 |
| 1284 | NM_001778 | 1260 |
| 1285 | NM_001752 | 1261 |
| 1286 | AK056410 | 1263 |
| 1287 | BC006386 | 1264 |

**Table 19. Additional Human Protein BMNs**

| **SEQ ID NO:** | **Accession Number** | **Table 16 SEQ ID NO:** | **Table 18 SEQ ID NO:** |
|---|---|---|---|
| 1288 | P05023 | 1252 | 1276 |
| 1289 | P26599 | 1253 | 1277 |
| 1290 | P26599 | 1254 | 1278 |
| 1291 | P52565 | 1255 | 1279 |
| 1292 | P26599 | 1256 | 1280 |
| 1293 | P14618 | 1257 | 1281 |
| 1294 | P30049 | 1258 | 1282 |
| 1295 | Q10567 | 1259 | 1283 |
| 1296 | P09326 | 1260 | 1284 |
| 1297 | P04040 | 1261 | 1285 |
| 1298 | Q8WUJ5 | 1263 | 1286 |
| 1299 | Q14296 | 1264 | 1287 |

### Example 1

### Spinal nerve ligation and artemin treatment

Male Srague-Dawley rats were subjected to unilateral spinal nerve ligation (SNL) performed according to the procedure of Kim and Chung (1992) Pain, 50:355-365. Rats with motor deficiency were excluded. The L₅ and L₆ spinal nerves of anesthetized rats were exposed and tightly ligated with 4-0 silk sutures. Sham surgery was identical but without actual ligation.

Rat artemin (113 amino acids; SEQ ID N0:1237) was isolated and refolded from E. coli inclusion bodies and purified to > 98% homogeneity (Gardell et al. (2003) Nature Med., 9(11):1383-1389). (The amino acid sequence of human artemin is set out in SEQ ID N0:1238). The purified artemin migrated as a reducible dimer by SDS-PAGE and eluted as a single peak (24 kDa) by size exclusion chromatography and by reverse phase HPLC. The purified product was confirmed to contain the characteristic cysteine knot disulfide pattern seen in GDNF, and to be fully active in vitro by assaying receptor binding, cell-based c-RET kinase activation (Sanicola et al. (1997) Proc. Natl. Acad. Sci. USA, 94:6238-6243) and sensory neuronal survival. Artemin (1 mg/kg) was injected subcutaneously on days 3, 5, 7, 10, 12 and 14 following spinal nerve ligation surgery.

### Behavioral assays

Hyperalgesia to thermal stimulation was assessed as described by Hargreaves et al. (1988) Pain, 32:77-88. Latency to withdrawal of a hindpaw in response to noxious radiant heat was determined. A maximal cut-off of 40 sec prevented tissue damage.

Tactile withdrawal thresholds were measured by probing the hindpaw with 8 calibrated von Frey filaments (Stoelting, Wood Dale, Illinois) (0.41 g to 15 g). Each filament was applied to the plantar surface of the hindpaw using the up-down method as described by Chaplan et al. (1994) J. Neurosci. Methods, 53, 55-63. Withdrawal threshold was determined by sequentially increasing and decreasing the stimulus strength and calculated with a Dixon non-parametric test (Dixon (1980) Ann. Rev. Pharmacol. Toxicol., 20:441-462).

Following behavioral confirmation of nerve ligation-induced tactile and thermal hyperalgesia, and efficacy of artemin on neuropathic pain behavior, skin samples were collected on day 14 post-spinal nerve ligation (following artemin injection and behavioral testing) from L4 dermatomes for subsequent gene expression profiling. The skin was shaved to remove as much hair as possible, and 12 skin samples in total were collected and snap-frozen, comprising triplicates from each of 4 groups of rats: (1) vehicle treated + SNL injury (ipsilateral to injury), (2) vehicle treated + SNL injury (contralateral to injury), (3) artemin treated + SNL injury (ipsilateral to injury), and (4) artemin treated + SNL injury (contralateral to injury).

### Total RNA Purification

Snap frozen skin samples were homogenized using an Ultra-Turrax T8 (IKA-Werke, Staufen, Germany) in TRIzol^{™} reagent (Invitrogen Life Technologies, Carlsbad, CA) according to manufacturer's protocol. 100 µg of total RNA was further purified using an RNeasy^{™} Mini column (Qiagen, Valencia, CA) according to manufacturer's protocol.

### Probe labeling, hybridization and scanning

The mRNA from skin biopsies samples was profiled on Affymetrix Rat Genome U34A, U34B, and U34C GeneChips^{™} probe arrays. These arrays contain more than 24,000 mRNA transcripts from gene and EST sequences found in Build 34 of the UniGene^{™} Database with additional full-length sequences from GenBank^{™} 110. GeneChip^{™} probe arrays are made by synthesizing oligonucleotide probes directly onto a glass surface. Each 25mer oligonucleotide probe is uniquely complementary to a gene, with approximately 16 pairs of oligonucleotide probes used to measure the transcript level of each of the genes represented in the array.

Sample labeling, hybridization, and staining were carried out according to the Eukaryotic Target Preparation protocol in the Affymetrix^{™} Technical Manual (701021 rev 1) for GeneChip^{™} Expression Analysis (Affymetrix, Santa Clara, CA). In summary, 5 µg of purified total RNA was used in a 20 µL first strand reaction with 200 U SuperScript^{™} II (Invitrogen Life Technologies, Carlsbad, CA) and 0.5 µg (dT)-T7 primer (SEQ ID N0:1239) in 1x first strand buffer (Invitrogen, Carlsbad, CA) with a 42°C incubation for 1 hour. Second strand synthesis was carried out by the addition of 40 U of *E. coli* DNA Polymerase, 2 U of E. coli RNase H, 10 U of *E. coli* DNA ligase in 1x second strand buffer (Invitrogen) followed by incubation at 16°C for 2 hrs. The second strand synthesis reaction was purified using the GeneChip^{™} Sample Cleanup Module according to the manufacturer's protocol (Affymetrix). The purified cDNA was amplified using BioArray^{™} high yield RNA transcription labeling kit (Enzo Life Sciences, Parmingdale, NY) according to manufacturer's protocol to produce 70-120 µg of biotin labeled cRNA (compliment RNA). Rat Genome U34 A, B, and C GeneChip^{™} probe arrays were pre-hybridized in a GeneChip^{™} Hybridization Oven 640 (Affymetrix) according to the manufacturer's protocol. Fifteen µg of labeled cRNA was fragmented in 30 µL 1x fragmentation buffer 100 mM KOAc, 30 mM MgOAc at 95°C for 35 minutes. The fragmented labeled cRNA was resuspended in 300 µL 1x hybridization buffer containing 100 mM MES, 1 M Na+, 20 mM EDTA, 0.01 % Tween^{™} 20, 0.5 mg/mL acetylated BSA, 0.1 mg/mL herring sperm DNA, control oligo B2, and control transcripts bioB 1.5 pM, bioC 5 pM, bioD 25 pM, and cre 100 pM, and hybridized to GeneChip^{™} probe arrays according to manufacturer's protocol (Affymetrix, Santa Clara, CA). The hybridized GeneChip® probe arrays were washed and stained using streptavidin-phycoerythrinin (Molecular Probes, Eugene, OR) and amplified with biotinylated anti-streptavidin (Vector Laboratories, Burlingame, CA) (Sigma, Saint Louis, MO) GeneChip^{™} Fluidics Station 400 (Affymetrix) using an antibody amplification protocol. The GeneChip^{™} probe arrays were scanned using GeneArray^{™} scanner (Hewlett Packard, Corvallis, OR).

### Data analysis

Two independent analysis approaches (Rosetta Resolver^{™} and a proprietary permutation-based Bayesian statistical model) were used to identify bio- and surrogate markers.

The following analysis techniques were performed using Rosetta Resolver^{™} software (Rosetta Biosoftware, Kirkland, WA).

The triplicate samples were considered a single group for ANOVA analyses. The comparisons of interest include the following:
1) Vehicle-treated vs. artemin-treated contralateral dermatomes;
2) Vehicle-treated vs. artemin-treated ipsilateral dermatomes;
3) Contralateral vs. ipsilateral vehicle-treated dermatomes;
4) Contralateral vs. ipsilateral artemin-treated dermatomes.

A gene list was generated based on those genes whose expression level was found to be significantly different between groups (p ≤ 0.01). These genes were subsequently tested for significance (p ≤ 0.01) in fold-change values. The final gene list for each of the 4 comparisons included those genes that passed both criteria. Agglomerative hierarchical clustering techniques (heuristic criteria = average link, similarity measure = Euclidean distance, intensity/Z-score used for clustering) ensured that these final gene lists differentiated well the two populations in each comparison from each other.

Permutation-based Bayesian Analysis was performed as follows. For all genes, a permutation based approach was used to generate distributions of log ratios of the expression intensity values for all possible pairwise within group (between replicates) and between group comparisons of the samples.

For example, the 3 replicate rats generated 3 within-group pairwise comparisons for each of the 4 treatment scenarios outlined above. In this way, a total of 12 within-group log ratios and 9 between-group log ratios for the 6 possible between-group comparisons were generated (Table 14). This was done for the A, B, and C chips.

**Table 14.**

| Comparison type | Group 1 | Group 2 | No. of pairwise comparisons |
|---|---|---|---|
| Between group | Vehicle-treated, ipsilateral (3 replicate rats) | Vehicle-treated, contralateral (3 replicate rats) | 9 |
| Between group | artemin-treated, ipsilateral (3 replicate rats) | artemin-treated, contralateral (3 replicate rats) | 9 |
| Between group | artemin-treated, ipsilateral (3 replicate rats) | Vehicle-treated, ipsilateral (3 replicate rats) | 9 |
| Between group | artemin-treated, ipsilateral (3 replicate rats) | Vehicle-treated, contralateral (3 replicate rats) | 9 |
| Between group | Vehicle-treated, Ipsilateral (3 replicate rats) | artemin-treated, contralateral (3 replicate rats) | 9 |
| Between group | artemin-treated, contralateral (3 replicate rats) | Vehicle-treated, contralateral (3 replicate rats) | 9 |
| Within group | Vehicle-treated, ipsilateral (3 replicate rats) | Vehicle-treated, ipsilateral (3 replicate rats) | 3 |
| Within group | Vehicle-treated, contralateral (3 replicate rats) | Vehicle-treated, contralateral (3 replicate rats) | 3 |
| Within group | artemin-treated, ipsilateral (3 replicate rats) | artemin-treated, contralateral (3 replicate rats) | 3 |
| Within group | artemin-treated, contralateral (3 replicate rats) | artemin-treated, contralateral (3 replicate rats) | 3 |

All ratio calculations were performed using the Affymetrix^{™} MAS5 application that summarizes the ratios of background corrected intensities (perfect match minus the mismatch intensity values) using an Affymetrix^{™} proprietary error model described in Affymetrix Microarray Suite User's guide Version 5.0 (2001). Default parameters were used to quantify signal intensities (Alpha1=0.04; Alpha2=0.06; Tau=0.015; Noise (RawQ)=2.800; Scale Factor (SF)=1.000 Norm Factor (NF)=1.000; Gamma1 L=0.0025; Gamma1 H=0.0025; Gamma2L=0.003; Gamma2H=0.003; Perturbation =1.1). The summarized signal log ratios with their associated P values were exported for statistical analysis.

The prior distribution of the log ratios were used to update the P values (posterior probability) of the between group comparison log ratios. Genes with between group log ratio distributions that significantly (p < 0.05) differed from the within group distribution of log ratios were selected as differentially expressed genes. The summary log ratio for any comparison was estimated as an error-weighted mean of all the permuted log ratios in that group.

308 genes that are affected by spinal nerve ligation injury (vehicle-treated ipsilateral vs. contralateral dermatomes) and that therefore correlate with neuropathic pain behavior are listed in Table 2.

To identify surrogate markers of a rtemin neurotrophic activity, genes with specific profiles of interest (e.g., genes that were up-regulated after injury and then down-regulated to normal levels with administration of artemin) were found by intersecting the lists of genes comparing contralateral vs. ipsilateral vehicle-treated dermatomes and vehicle-treated vs. artemin-treated ipsilateral dermatomes. 107 surrogate markers of artemin neurotrophic activity thus identified are listed in Table 6.

To identify biomarkers of artemin's in vivo biological activity, genes in common on the lists comparing vehicle-treated vs. artemin-treated contralateral dermatomes and vehicle-treated vs. artemin-treated ipsilateral dermatomes were identified. Genes were then identified that are regulated in the same direction by artemin in the contralateral and ipsilateral dermatomes. 49 biomarkers of artemin biological activity were thus identified and are listed in Table 10. Fig. 7 shows an example of a BMN that has not been confirmed by TaqMan^{™} analysis.

To confirm the validity of surrogate markers and biomarkers, 25 preferred surrogate markers of neurotrophic activity and 5 preferred biomarkers were used for sequence analysis to validate the existence of transcripts. The sequence analysis included a BLAST^{™} search of the Affymetrix^{™} target sequence against the rat genomic sequence. The genomic locus was then examined for the existence of exons, ESTs, and predicted transcripts. The genes are prioritized based on transcript evidence and subjected to TaqMan^{™} validation as described below (see, also, Holland et al. (1991). Proc. Natl. Acad. Sci. USA, 88:7276-7280).

### TaqMan^{™} Analysis

Trizol^{™} (Invitrogen) purified rat skin RNA was further repurified using an RNeasy^{™} Mini kit (Qiagen) according to the manufacturer's protocol. The RNA was digested with Amplification Grade Deoxyribonuclease 1 (Invitrogen) to remove any contaminating DNA, and was subsequently used as a template for cDNA synthesis with a High-Capacity cDNA Archive Kit (Applied Biosystems). The resulting cDNA was used as the PCR template for TaqMan^{™} analysis.

The "TaqMan MGB Probe and Primer Design" function of Primer Express 1.5 software (Applied Biosystems) was used to generate primer and probe sequences for Affymetrix target sequences (for example, see Table 15 for rc_AA818804_at RG-U34C, rc_aa818120_at RG-U34C, and X14812_at RG-U34A).

**Table 15.**

| AffyID^{™} | Marker SEQ ID NO: | Amplicon SEQ ID NO: | Forward primer SEQ ID NO: | Reverse primer SEQ ID NO: | Probe SEQ ID NO: |
|---|---|---|---|---|---|
| rc_AA818120_at RG-U34C | 31,808 | 1240 | 1241 | 1242 | 1243 |
| rc_AA818804_at RG-U34C | 18,799 | 1244 | 1245 | 1246 | 1247 |
| X14812_at RG-U34A | 13 813 | 1248 | 1249 | 1250 | 1251 |

Oligomers spanning the PCR amplicon, plus an additional 10 bp on the 5' and 3' ends of each gene were also synthesized. Primers and 6FAM-labeled probes were synthesized by Applied Biosystems, and set up in reactions with the cDNA templates according to standard methods. Reactions were carried out in an ABI Prism^{™} 7700 Sequence Detector using the default conditions, and the data was analyzed using Sequence Detection Software v1.9.1 (Applied Biosystems). Simultaneous PCR reactions were carried out using a 10-fold dilutions series of the amplicon oligomers to generate a standard curve for each primer and probe set. Cycle Threshold (Ct) values for each experimental reaction were compared to the amplicon standard curve and relative quantities of message were determined. The cDNA samples were also analyzed with TaqMan^{™} Rodent GAPDH Control Reagents (Applied Biosystems) to determine the amount of GAPDH message in each sample. The samples were normalized by dividing the signal for each of the surrogate marker genes by the signal obtained with the GAPDH control. The results are shown in Figs. 1-6.

The expression patterns of the genes shown in Figs. 1-6 parallel the results of the Affymetrix analysis. All of these genes are expressed at a low level in the uninjured state (vehicle/contralateral and artemin/contralateral), are up-regulated in the injured state (vehicle/ipsilateral), and are at least partially normalized following artemin treatment (artemin/ipsilateral). The expression profiles are consistent with these genes acting as surrogate markers of artermin activity in the rat spinal nerve ligation model.

### Example 2: Determination of Artemln Biomarkers of Exposure

### Exposure biomarker samples

Uninjured male Sprague-Dawley rats were dosed with artemin or vehicle and L4 dermatomes were subsequently processed as described in Example 1. Eighteen samples were generated corresponding to left and right L4 dermatomes from triplicate animals of the following groups: (1) uninjured + artemin treated, (2) uninjured + vehicle treated, (3) naive.

### Data analysis

Two independent analysis approaches (Rosetta Resolver^{™} and Affymetrix/MAS5.0^{™}) were used to identify artemin exposure biomarkers.

The following analysis techniques were performed using Rosetta Resolver^{™} software (Rosetta Biosoftware, Kirkiand, WA). The left and-right triplicate samples were considered a single group for ANOVA analyses. The comparisons of interest include the following:
1) naive vs. vehicle-treated uninjured dermatomes;
2) naive vs. artemin-treated uninjured dermatomes; and
3) vehicle-treated vs. artemin-treated uninjured dermatomes.

A gene list was generated based on those genes whose expression level was found to be significantly different between groups (p ≤ 0.01). These genes were subsequently tested for significance (p ≤ 0.05) in fold-change values and magnitude of fold change (-2 ≥ Z-score ≥ 2). The final gene list for each of the comparisons included those genes that passed all three criteria.

To identify biomarkers of artemin's in vivo biological activity, genes in common on the lists comparing naive vs. artemin-treated dermatomes and vehicle-treated vs. artemin-treated dermatomes were identified. A subset of these genes, which were not altered between naive and vehicle treatments, were subsequently identified as biomarkers of artemin exposure (BMNs).

Affymetrix/MAS5^{™} analysis was performed as follows. This strategy involved first identifying AffyIDs that were differentially expressed either: (i) between the left and right sides of animals or (ii) in identical treatments across animals, thereby indicating inter-animal heterogeneity. Next, all treatments were compared to understand the effects of systemic artemin administration on gene expression in skin. AffyID^{™}'s found in (i) or (ii) above or determined to be changed due to vehicle treatment only were eliminated from final consideration.

All possible pair-wise ratios of expression intensity values were built using the Affymetrix® MAS5 software that uses background corrected intensities (perfect match minus the mismatch intensity values) using an Affymetrix® proprietary error model described in Affymetrix Microarray Suite User's guide Version 5.0. 2001. For pair-wise ratios, MAS5 determines whi ch AffyIDs are changed (increased or decreased "I/D") or not changed ("NC") using a Wilcoxon Signed Rank Test.

More than half of the ratios representing comparisons (i) and (ii) above had to exhibit "NC"; otherwise, the AffylD was removed from consideration. Twenty-three total ratios were considered over all three chips for the left vs right comparison, and 358 AffyID^{™}'s failed. Twenty-seven total ratios were considered over all three chips for the inter-animal comparison, and 1,375 AffyID^{™}'s failed.

For the comparison between treatments, 27 ratios were built and considered for each comparison (9 per chip). At least 8 of the 9 ratios had to be consistently increased or Decreased to be considered for further analysis.

The gene lists comparing artemin treatment with Naïve and Vehicle treatments were intersected, and 10 AffyID^{™}'s were found to be candidates for artemin exposure markers.

When the intersection of these two analyses was made, 2 AffyIDs were found in common. The 13 genes that result from the union of these two analyses are listed as biomarkers of artemin exposure in Table 16.

All references to nucleotide sequences should be understood to encompasses their sequences complementary to a given sequence. All publications and patents and sequences cited in this disclosure by their accession numbers are incorporated by reference in their entirety. To the extent the material incorporated by reference contradicts or is inconsistent with the present specification, the specification will supercede any such material.

The material submitted herewith on the CD-ROM entitled "Surrogate Markers of Neuropathic Pain," containing file surrmarkers021705.ST25.txt, size on disk 4,689,920 bytes, created on February 17, 2004, is hereby incorporated by reference.

### Further aspects of the invention are:

A) A method of identifying a surrogate marker of neuropathic pain in a mammal, comprising:
   (a) obtaining a first skin biopsy sample under conditions of neuropathic pain;
   (b) obtaining a second skin biopsy sample under conditions of substantially no neuropathic pain;
   (c) preparing tissue extracts from the first and the second samples; and
   (d) determining an amount of at least one nucleic acid or protein in the tissue extracts;
   wherein a difference between the amount of the nucleic acid or the protein in the first sample and the amount of the same nucleic acid or protein in the second sample indicates that the nucleic acid or the protein is a surrogate marker of neuropathic pain.
The method A) above, wherein the amount of the nucleic acid or the protein in the first sample differs at least 2-fold from the amount of the same nucleic acid or protein in the second sample.
The method A) above, wherein the first and the second samples are obtained from the same mammal.
   The method A) above, wherein the mammal is a rodent.
   The method A) above, wherein the mammal is a human.
   The method A) above, wherein the nucleic acid or protein is muscle-specific.
B). A method of evaluating the level of neuropathic pain in a mammal, comprising:
   (a) obtaining a first skin biopsy sample under conditions of neuropathic pain;
   (b) obtaining a second skin biopsy sample under conditions of substantially no neuropathic pain;
   (c) preparing tissue extracts from the first and the second samples; and
   (d) determining an amount of at least one nucleic acid or protein in the tissues, the nucleic acid or the protein being a surrogate marker of neuropathic pain;
   wherein a difference between the amount of the nucleic acid or the protein in the first sample and the amount of the same nucleic acid or protein in the second sample indicates the level of neuropathic pain.
   The method B) above, wherein the amount of the nucleic acid or the protein in the first sample differs at least 2-fold from the amount of the same nucleic acid or protein in the second sample.
   The method B) above, wherein the first and the second samples are obtained from the same mammal.
   The method B) above, wherein the mammal is a rodent.
   The method B) above, wherein the nucleic acid comprises a nonredundant subsequence of any one of the nucleotide sequences of SEQ ID NOs:1-308.
   The method B) above, wherein the nucleic acid comprises a nonredundant subsequence of any one of the nucleotide sequences of SEQ ID NOs:1-42.
   The method B) above wherein the protein comprises a nonredundant subsequence of any one of amino acid sequences of SEQ ID NOs:309-470.
   The method B) above, wherein the protein comprises a nonredundant subsequence of any one of amino acid sequences of SEQ ID NOs:309-333.
   The method B) above, wherein the mammal is a human.
   The method B) above, wherein the nucleic add comprises a nonredundant subsequence of any one of the nucleotide sequences of SEQ ID NOs:471-630.
   The method B) above, wherein the nucleic acid comprises a nonredundant subsequence of any one of the nucleotide sequences of SEQ ID NOs:471-493.
   The method B) above, wherein the protein comprises a nonredundant subsequence of any one of amino acid sequences of SEQ ID NOs:631-790.
   The method B) above, wherein the protein comprises a nonredundant subsequence of any one of amino acid sequences of SEQ ID NOs:631-653.
   The method B) above, wherein the surrogate marker is muscle-specific.
C) A method of evaluating the effect of a compound or composition on the level of neuropathic pain in a mammal, comprising:
   (a) administering the compound or composition to the mammal having neuropathic pain;
   (b) obtaining at least one skin biopsy sample from the mammal;
   (c) preparing a tissue extract from the skin biopsy sample; and
   (d) determining an amount of at least one nucleic acid or protein in the tissue extract, the nucleic acid or the protein being a surrogate marker of neuropathic pain;
   wherein a difference in the amount of the nucleic acid or protein determined in step (d) and the amount of the same nucleic acid or protein expressed in the absence of the compound or composition indicates the level of efficacy of the compound or composition on neuropathic pain.
   The method C) above, wherein the amount determined in step (d) that differs at least 2-fold from the amount of the same nucleic acid or protein expressed in the absence of the compound or composition.
   The method C) above wherein the compound or composition is a neurotrophic agent.
   The method C) above, wherein the neurotrophic agent belongs to the glial cell-derived neurotrophic factor (GDNF) family.
   The method C) above, wherein the neurotrophic agent is artemin.
   The method C) above, wherein the mammal is a rodent.
   The method C) above, wherein the nucleic acid comprises a nonredundant subsequence of any one of the nucleotide sequences of SEQ ID NOs:791-897.
   The method C) above, wherein the nucleic acid comprises a nonredundant subsequence of any one of the nucleotide sequences of SEQ ID N0s:791-814.
   The method C) above, wherein the protein comprises a nonredundant subsequence of any one of amino acid sequences of SEQ ID N0s:898-962.
   The method C) above, wherein the protein comprises a nonredundant subsequence of any one of amino acid sequences of SEQ ID NOs:898-914.
   The method C) above, wherein the mammal is a human.
   The method C) above, wherein the nucleic acid comprises a nonredundant subsequence of any one of the nucleotide sequences of SEQ ID NOs:963-1038.
   The method C) above, wherein the nucleic acid comprises a nonredundant subsequence of any one of the nucleotide sequences of SEQ ID N0s:963-979.
   The method C) above, wherein the protein comprises a nonredundant subsequence of any one of amino acid sequences of SEQ ID NOs:1039-1114.
   The method C) above, wherein the protein comprises a nonredundant subsequence of any one of amino acid sequences of SEQ ID NOs:1039-1055.
   The method C) above, wherein the nucleic acid or protein is muscle-specific.
D) method of identifying a biomarker of biological activity of a neurotrophic agent, comprising:
   (a) administering the agent to a mammal;
   (b) obtaining at least one skin biopsy sample from the mammal;
   (c) preparing a tissue extract from the skin biopsy sample; and
   (d) determining an amount of at least one nucleic acid or protein in the tissue;
   wherein a difference in the amount of the nucleic acid or protein determined in step (d) and the amount of the same nucleic acid or protein expressed in the absence of the agent indicates that the nucleic add or the protein is a biomarker of in vivo biological activity of the agent.
   The method D) above, wherein the amount determined in step (d) differs at least 2-fold from the amount of the same nucleic acid or protein expressed in the absence of the agent.
   The method D) above, wherein the neurotrophic agent belongs to the glial cell-derived neurotrophic factor (GDNF) family.
   The method D) above, wherein the neurotrophic agent is artemin.
   The method D) above, wherein the nucleic acid or protein is muscle-specific.
E) A method of evaluating in vivo biological activity of a neurotrophic agent, comprising:
   (a) administering the agent to a mammal;
   (b) obtaining at least one skin biopsy sample from the mammal;
   (c) preparing a tissue extract from the skin biopsy sample; and
   (d) determining an amount of at least one nucleic acid or protein in the tissue extract;
   wherein a difference in the amount of the nucleic acid or protein determined in step (d) and the amount of the same nucleic acid or protein expressed in the absence of the agent indicates that the agent is biologically active.
   The method E) above, wherein the amount determined in step (d) differs at least 2-fold from the amount of the same nucleic acid or protein expressed in the absence of the agent.
   The method E) above, wherein the nucleic acid or the protein is a surrogate marker of neuropathic pain.
   The method E) above, wherein the nucleic acid of the protein is a surrogate marker of neurotrophic activity of the agent.
   The method E) above, wherein the neurotrophic agent belongs to the glial cell-derived neurotrophic factor (GDNF) family.
   The method E) above, wherein the neurotrophic agent is artemin.
   The method E) above, wherein the mammal is a rodent.
   The method E) above, wherein the nucleic acid comprises a nonredundant subsequence of any one of the nucleotide sequences of SEQ ID NOs:1115-1163.
   The method E) above, wherein the nucleic acid comprises a nonredundant subsequence of any one of the nucleotide sequences of SEQ ID NOs:1252-1264.
   The method E) above, wherein the nucleic acid comprises a nonredundant subsequence of any one of the nucleotide sequences of SEQ ID NOs:1115-1120.
   The method E) above, wherein the nucleic acid comprises a nonredundant subsequence of any one of the nucleotide sequences of SEQ ID NOs:1252-1256.
   The method E) above, wherein the protein comprises a nonredundant subsequence of any one of amino acid sequences of SEQ ID NOs:1164-1178.
   The method E) above, wherein the protein comprises a nonredundant subsequence of any one of amino acid sequences of SEQ ID NOs:1265-1274.
   The method E) above, wherein the protein comprises a nonredundant subsequence of any one of amino acid sequences of SEQ ID NOs:1164-1166.
   The method E) above, wherein the protein comprises a nonredundant subsequence of any one of amino acid sequences of SEQ ID NOs:1265-1269.
   The method E) above, wherein the mammal is a human.
   The method E) above, wherein the nucleic acid comprises a nonredundant subsequence of any one of the nucleotide sequences of SEQ ID NOs:1179-1207.
   The method E) above, wherein the nucleic acid comprises a nonredundant subsequence of any one of amino acid sequences of SEQ ID NOs:1276-1287.
   The method E) above, wherein the nucleic acid comprises a nonredundant subsequence of any one of the nucleotide sequences of SEQ ID NOs:1179-1182.
   The method E) above, wherein the nucleic acid comprises a nonredundant subsequence of any one of the nucleotide sequences of SEQ ID NOs:1276-1280.
   The method E) above, wherein the protein comprises a nonredundant subsequence of any one of amino acid sequences of SEQ ID NOs:1208-1236.
   The method E) above, wherein the protein comprises a nonredundant subsequence of any one of amino acid sequences of SEQ ID NOs:1288-1299.
   The method E) above, wherein the protein comprises a nonredundant subsequence of any one of amino acid sequences of SEQ ID NOs:1208-1211.
   The method E) above, wherein the protein comprises a nonredundant subsequence of any one of amino acid sequences of SEQ ID NOs:1288-1292.
   The method E) above, wherein the nucleic acid or protein is muscle-specific.
F) A method of evaluating the effect of artemin on the level of neuropathic pain in a mammal, comprising:
   (a) administering artemin to a mammal having neuropathic pain;
   (b) obtaining at least one skin biopsy sample from the mammal;
   (c) preparing a tissue extract from the skin biopsy sample; and
   (d) determining an amount of at least one nucleic acid in the tissue extract, the nucleic acid comprising a nonredundant subsequence of any one of the nucleotide sequences of SEQ ID NOs:791-814;
   wherein a difference in the amount of the nucleic acid or protein determined in step (d) and the amount of the same nucleic add or protein expressed in the absence of the compound or composition indicates the level of efficacy of the compound or composition on neuropathic pain.
   The method F) above, wherein the amount determined in step (d) that differs at least 2-fold from the amount of the same nucleic acid or protein expressed in the absence of artemin.
   The method F) above, wherein the mammal is a rodent.
   The method F) above, wherein neuropathic pain is caused by a spinal nerve injury.
G). The method F) above, comprising obtaining a second skin biopsy sample under conditions of substantially no neuropathic pain.
H). The method G) above, wherein the first and the second samples are obtained from the same mammal.
   The method H) above, wherein the first skin biopsy sample is obtained from a first site contralateral to the spinal nerve injury, and a second skin biopsy sample is obtained from a second site ipsilateral to the spinal nerve injury.
   The method F) above, wherein the nucleic acid or protein is muscle-specific.

## Claims

1. An in vitro method of identifying a surrogate marker of neuropathic pain in a mammal, comprising:
(a) preparing tissue extracts from a first skin biopsy sample obtained under conditions of neuropathic pain and a second skin biopsy sample obtained under conditions of substantially no neuropathic pain; and
(b) determining an amount of at least one nucleic acid or protein in the tissue extracts;
wherein a difference between the amount of the nucleic acid or the protein in the first sample and the amount of the same nucleic acid or protein in the second sample indicates that the nucleic acid or the protein is a surrogate marker of neuropathic pain.

2. An in vitro method of evaluating the level of neuropathic pain in a mammal, comprising:
(a) preparing tissue extracts from a first skin biopsy sample obtained under conditions of neuropathic pain and a second skin biopsy sample obtained under conditions of substantially no neuropathic pain; and
(b) determining an amount of at least one nucleic acid or protein in the tissues, the nucleic acid or the protein being a surrogate marker of neuropathic pain;
wherein a difference between the amount of the nucleic acid or the protein in the first sample and the amount of the same nucleic acid or protein in the second sample indicates the level of neuropathic pain.

3. The method of claims 1 or 2, wherein the amount of the nucleic acid or the protein in the first sample differs at least 2-fold from the amount of the same nucleic acid or protein in the second sample.

4. The method of any one of the preceding claims, wherein the nucleic acid comprises a nonredundant subsequence of any one of the nucleotide sequences of SEQ ID NOs:1-308.

5. The method of claim 4, wherein the nucleic acid comprises a nonredundant subsequence of any one of the nucleotide sequences of SEQ ID NOs:1-42.

6. The method of any one of claims 1 to 3, wherein the protein comprises a nonredundant subsequence of any one of amino acid sequences of SEQ ID NOs:309-470.

7. The method of claim 6, wherein the protein comprises a nonredundant subsequence of any one of amino acid sequences of SEQ ID NOs:309-333.

8. The method of claims 1 to 3, wherein the nucleic acid comprises a nonredundant subsequence of any one of the nucleotide sequences of SEQ ID NOs:471-630.

9. The method of claim 8, wherein the nucleic acid comprises a nonredundant subsequence of any one of the nucleotide sequences of SEQ ID NOs:471-493.

10. The method of any one of claims 1-3, wherein the protein comprises a nonredundant subsequence of any one of amino acid sequences of SEQ ID NOs:631-790.

11. The method of claim 10, wherein the protein comprises a nonredundant subsequence of any one of amino acid sequences of SEQ ID NOs:631-653.

12. The method of any one of the preceding claims, wherein the surrogate marker is muscle-specific.

13. An in vitro method of evaluating the effect of a compound or composition on the level of neuropathic pain in a mammal, comprising:
(a) preparing a tissue extract from at least one skin biopsy sample from a mammal having neuropathic pain, to which the compound or composition has been administered ; and
(b) determining an amount of at least one nucleic acid or protein in the tissue extract, the nucleic acid or the protein being a surrogate marker of neuropathic pain;
wherein a difference in the amount of the nucleic acid or protein determined in step (b) and the amount of the same nucleic acid or protein expressed in the absence of the compound or composition indicates the level of efficacy of the compound or composition on neuropathic pain.

14. The method of claim 13, wherein the amount determined in step (b) differs at least 2-fold from the amount of the same nucleic acid or protein expressed in the absence of the compound or composition.

15. The method of claims 13 or 14, wherein the nucleic acid comprises a nonredundant subsequence of any one of the nucleotide sequences of SEQ ID NOs:791-897.

16. The method of claim 15, wherein the nucleic acid comprises a nonredundant subsequence of any one of the nucleotide sequences of SEQ ID NOs:791-814.

17. The method of claims 13 or 14, wherein the protein comprises a nonredundant subsequence of any one of amino acid sequences of SEQ ID NOs:898-962.

18. The method of claim 17, wherein the protein comprises a nonredundant subsequence of any one of amino acid sequences of SEQ ID NOs:898-914.

19. The method of claims s 13 or 14, wherein the nucleic acid comprises a nonredundant subsequence of any one of the nucleotide sequences of SEQ ID NOs:963-1038.

20. The method of claim 19, wherein the nucleic acid comprises a nonredundant subsequence of any one of the nucleotide sequences of SEQ ID NOs:963-979.

21. The method of claims 13 or 14, wherein the protein comprises a nonredundant subsequence of any one of amino acid sequences of SEQ ID NOs:1039-1114.

22. The method of claim 21, wherein the protein comprises a nonredundant subsequence of any one of amino acid sequences of SEQ ID NOs:1039-1055.

23. The method of any one of claims 13-22, wherein the compound or composition is a neurotrophic agent.

24. An in vitro method of identifying a biomarker of biological activity of a neurotrophic agent, comprising:
(a) preparing a tissue extract from at least one skin biopsy sample obtained from a mammal, to which the agent has been administered; and
(b) determining an amount of at least one nucleic acid or protein in the tissue;
wherein a difference in the amount of the nucleic acid or protein determined in step (b) and the amount of the same nucleic acid or protein expressed in the absence of the agent indicates that the nucleic acid or the protein is a biomarker of in vivo biological activity of the agent.

25. An in vitro method of evaluating in vivo biological activity of a neurotrophic agent, comprising:
(a) preparing a tissue extract from at least one skin biopsy sample from a mammal, to which the agent has been administered; and
(b) determining an amount of at least one nucleic acid or protein in the tissue extract;
wherein a difference in the amount of the nucleic acid or protein determined in step (b) and the amount of the same nucleic acid or protein expressed in the absence of the agent indicates that the agent is biologically active.

26. The method of claims 24 or 25, wherein the amount determined in step (b) differs at least 2-fold from the amount of the same nucleic acid or protein expressed in the absence of the agent.

27. The method of any one of claims 24-26, wherein the nucleic acid or the protein is a surrogate marker of neuropathic pain or of neurotrophic activity of the agent.

28. The method of any one of claims 24-27, wherein the nucleic acid comprises a nonredundant subsequence of any one of the nucleotide sequences of SEQ ID NOs:1115-1163 or SEQ ID NOs: 1252-1264.

29. The method of claim 28, wherein the nucleic acid comprises a nonredundant subsequence of any one of the nucleotide sequences of SEQ ID NOs: 1115-1120.

30. The method of claim 28, wherein the nucleic acid comprises a nonredundant subsequence of any one of the nucleotide sequences of SEQ ID NOs: 1252-1256.

31. The method of any one of claims 24-27, wherein the protein comprises a nonredundant subsequence of any one of amino acid sequences of SEQ ID NOs:1 164-1178 or SEQ ID NOs:1265-1274.

32. The method of claim 31, wherein the protein comprises a nonredundant subsequence of any one of amino acid sequences of SEQ ID NOs:1164-1166.

33. The method of claim 31, wherein the protein comprises a nonredundant subsequence of any one of amino acid sequences of SEQ ID NOs:1265-1269.

34. The method of any one of claims 24-27, wherein the nucleic acid comprises a nonredundant subsequence of any one of the nucleotide sequences of SEQ ID NOs:1179-1207 or SEQ ID NOs: 1276-1287.

35. The method of claim 34, wherein the nucleic acid comprises a nonredundant subsequence of any one of the nucleotide sequences of SEQ ID NOs:1179-1182.

36. The method of claim 34, wherein the nucleic acid comprises a nonredundant subsequence of any one of the nucleotide sequences of SEQ ID NOs:1276-1280.

37. The method of any one of claims 24-27, wherein the protein comprises a nonredundant subsequence of any one of amimo acid sequences of SEQ ID NOs:1208-1236 or SEQ ID NOs:1288-1299.

38. The method of claim 37, wherein the protein comprises a nonredundant subsequence of any one of amino acid sequences of SEQ ID NOs:1208-1211.

39. The method of claim 37, wherein the protein comprises a nonredundant subsequence of any one of amino acid sequences of SEQ ID NOs:1288-1292.

40. The method of any one of claims 23-39, wherein the neurotrophic agent belongs to the glial cell-derived neurotrophic factor (GDNF) family.

41. The method of claim 40, wherein the neurotrophic agent is artemin.

42. The method of any one of the preceding claims, wherein the mammal is a rodent.

43. The method of any one of claims 1-41, wherein the mammal is a human.

44. The method of any one of the preceding claims, wherein neuropathic pain is caused by a spinal nerve injury.

45. The method of any one of the preceding claims, wherein the first and the second samples are obtained from the same mammal.

46. The method of claim 45, wherein the first skin biopsy sample is obtained from a first site contralateral to the spinal nerve injury, and a second skin biopsy sample is obtained from a second site ipsilateral to the spinal nerve injury.

47. The method of any one of the preceding claims, wherein the nucleic acid or protein is muscle-specific.
